# EUROPEAN PATENT APPLICATION

(11) **EP 4 357 365 A1**
(43) Date of publication of application: **24.04.2024**
(21) Application number: 21945436.0
(22) Date of filing: 16.06.2021
(51) Int. Cl.: C07K 16/40, A61K 39/395, C12N 5/10, C12N 15/13, C12N 15/63, A61P 35/00, A61K 45/00, A61K 35/17

(54) **ANTIBODY TARGETING AXL PROTEIN AND ANTIGEN BINDING FRAGMENT THEREOF, AND PREPARATION METHOD THEREFOR AND APPLICATION THEREOF**

(71) Applicant: Shanghai Sinobay Biotechnology Co., Ltd., Shanghai 201506 (CN)
(72) Inventor: XU, Jianqing, Shanghai 201506 (CN); ZHANG, Xiaoyan, Shanghai 201506 (CN); DING, Xiangqing, Shanghai 201506 (CN); LIAO, Qibin, Shanghai 201506 (CN)
(74) Representative: Uexküll & Stolberg
(86) International application number: PCT/CN2021/100263
(87) International publication number: WO 2022/261846

(57) **Abstract**

The present invention provides an antibody targeting an AXL protein or an antigen binding fragment thereof, and a preparation method therefor and use thereof. Further provided are an isolated polynucleotide encoding the antibody targeting the AXL protein or the antigen binding fragment thereof, and a vector comprising the isolated polynucleotide. The antibody targeting the AXL provided by the present invention can specifically bind to the AXL protein, can mediate ADCC to kill tumors, or have an anti-tumor effect as a target recognition domain of CART cells, and can be used for preventing or treating tumors, and hence it has a wide application prospect.

## Description

### Technical Field

The present invention belongs to the field of biological immunology and particularly relates to an antibody and an antigen binding fragment thereof capable of specifically binding to an AXL protein, and to a preparation method and use of the antibody and the antigen binding fragment thereof.

### Background Art

AXL is a 104-140 kDa transmembrane protein that belongs to the TAM subfamily of mammalian receptor tyrosine kinases (RTK) and has a transforming capability (Paccez et al., 2014). The AXL extracellular domain consists of a combination of two membrane-distal N-terminal immunoglobulin (Ig)-like domains (Ig1 and Ig2 domains) and two membrane-proximal fibronectin III-type (FNIII) repeats (FN1 and FN2 domains) (Paccez et al., 2014). The AXL protein is encoded by the gene AXL (UFO, ARK, Tyro7, or JTK11), and its receptor is a member of Tyro-3 family of tyrosine kinases. After binding to ligand Gas6 (homologous to anti-coagulation factor protein S, 70-kDa), it can transduce signals through PI-3K/Akt, Ras/Erk, and β-catenin/TCF to regulate the survival, proliferation, migration and adhesion of tumor cells. AXL is the first identified transforming gene in chronic myeloid leukemia (CML). Subsequent studies found that compared with normal tissues, AXL is activated and expressed in many kinds of tumor tissues, such as lung cancer, breast cancer, prostate cancer, thyroid cancer, endometrial cancer, ovarian cancer, and renal cancer, especially in highly invasive and highly metastatic basal-like and/or triple-negative breast cancer, metastatic lung cancer, and pancreatic cancer, and is involved in regulating epithelial-mesenchymal transition, angiogenesis, apoptosis, and immune regulation of tumor cells, promoting tumor metastasis and invasion, and thus leading to poor prognosis. However, there was no significant increase in non-tumor diseases. For example, no increase in soluble AXL (sAxl) was observed in the serum of patients suffering from chronic viral hepatitis, autoimmune hepatitis, cholestatic liver disease, or nonalcoholic fatty liver disease (CLD). However, there is a specific increase in sAxl concentration in the serum of patients with hepatocellular carcinoma (HCC), advanced fibrosis (stage F3), or cirrhosis (stage F4) with highly associated liver cancer. AXL can therefore also be used as a biomarker for the clinical diagnosis of tumors.

Recent studies have shown that the high expression of AXL protein is also closely related to the development of tumor drug resistance. In cancer cells resistant to chemotherapy and receptor tyrosine kinase inhibitor (TKI) treatment, such as EGFR inhibitor-refractory lung cancer, PI3K inhibitor-resistant head and neck cancer, anti-HER2 antibody-resistant breast cancer, sunitinib-resistant renal cancer, and ALK inhibitor-resistant neuroblastoma, the expression of AXL can be significantly increased. Inhibition of AXL expression can reverse the sensitivity of drug-resistant tumor cells to cytotoxic drugs and targeted inhibitors. Therefore, AXL kinase inhibitors have become a new strategy for cancer therapy. In addition, the only ligand of AXL, Gas6, is also highly expressed in various malignant tumors. Specifically blocking the interaction of AXL-Gas6 can effectively inhibit the migration, invasion, and drug resistance of tumor cells. Clinical studies have shown that AVB-500 can effectively inhibit the progression of platinum-resistant ovarian cancer by inhibiting the AXL/Gas6 signaling pathway. At present, this study has completed a phase I clinical trial in the United States and is about to enter a phase II registration clinical study.

Compared with other targets, antibodies targeting AXL can be rapidly and massively internalized, which helps to increase the number bound to antigenicity-related tumor target cell surfaces, decrease the number bound to non-target cells, increase the number entering target cells, and decrease the number entering non-target cells. Therefore, the development of antibody-drug conjugate (ADC), to which AXL specifically binds, has drawn attention in the field. Due to increased glycolysis, hypoxia, and inadequate tissue perfusion in the tumor microenvironment, the accumulation of large amounts of acidic metabolites renders the tumor microenvironment acidic, typically at a pH in the range of 5.8 to 7.0, most typically between 6.4 and 6.8. This acidic tumor microenvironment promotes tumor proliferation, invasion, migration, and immune escape, and presents challenges for tumor therapy. Therefore, it is urgently needed to develop more specific antibodies targeting AXL with high affinity, low immunogenicity, and good stability in the acidic tumor microenvironment, so as to improve the therapeutic effect of the tumor.

The data show that the antibody-drug conjugate (ADC) prepared by combining AXL monoclonal antibody and small molecule cytotoxic drug can enhance the targeting of the tumor and reduce toxic side effects. Compared with a fully or partially humanized antibody or an antibody fragment drug, ADC releases a highly active cytotoxic drug in tumor tissue to effectively inhibit tumor growth and metastasis. Clinical studies have shown that ADCs derived from existing AXL antibodies are prone to drug resistance due to their single targeting site towards tumor antigens, and their low blood half-life leads to increased toxicity. Therefore, there is a need for further development of optimized AXL antibody drugs.

### Summary of the Invention

Based on the deficiencies of the prior art, the main object of the present invention is to provide an antibody targeting the AXL protein with higher affinity, higher specificity, lower immunogenicity, and better stability under acidic conditions. The present invention also provides a preparation method and use of the antibody, and the AXL protein-targeting antibodies of the present invention can be used to detect and/or treat tumors.

On the one hand, the present invention provides an antibody or an antigen binding fragment thereof capable of specifically binding to an AXL protein, comprising:
(a) a heavy chain variable region (VH) comprising the following three complementary determining regions (CDR):
   (i) VH CDR1 consisting of a sequence of SEQ ID NO: 9, or a sequence having one or more amino acid substitutions, deletions, or additions (for example, 1, 2, or 3 amino acid substitutions, deletions, or additions) compared thereto,
   (ii) VH CDR2 consisting of a sequence of SEQ ID NO: 10, or a sequence having one or more amino acid substitutions, deletions, or additions (for example, 1, 2, or 3 amino acid substitutions, deletions, or additions) compared thereto, and
   (iii) VH CDR3 consisting of a sequence of SEQ ID NO: 11, or a sequence having one or more amino acid substitutions, deletions, or additions (for example, 1, 2, or 3 amino acid substitutions, deletions, or additions) compared thereto;
      and/or
(b) a light chain variable region (VL) comprising the following three complementary determining regions (CDR):
   (iv) VL CDR1 consisting of a sequence of SEQ ID NO: 12, or a sequence having one or more amino acid substitutions, deletions, or additions (for example, 1, 2, or 3 amino acid substitutions, deletions, or additions) compared thereto,
   (v) VL CDR2 consisting of a sequence of SEQ ID NO: 13, or a sequence having one or more amino acid substitutions, deletions, or additions (for example, 1, 2, or 3 amino acid substitutions, deletions, or additions) compared thereto, and
   (vi) VL CDR3 consisting of a sequence of SEQ ID NO: 14, or a sequence having one or more amino acid substitutions, deletions, or additions (for example, 1, 2, or 3 amino acid substitutions, deletions, or additions) compared thereto;

preferably, the substitution described in any one of (i)-(vi) is a conservative substitution; and
preferably, the VH of the antibody or the antigen binding fragment thereof comprises VH CDR1 as shown in SEQ ID NO: 9, VH CDR2 as shown in SEQ ID NO: 10, and VH CDR3 as shown in SEQ ID NO: 11; and the VL of the antibody or the antigen binding fragment thereof comprises: VL CDR1 as shown in SEQ ID NO: 12, VL CDR2 as shown in SEQ ID NO: 13, and VL CDR3 as shown in SEQ ID NO: 14.

On the one hand, the present invention provides an antibody or an antigen binding fragment thereof capable of specifically binding to an AXL protein, comprising:
(a) a heavy chain variable region (VH) comprising the following three complementary determining regions (CDR):
   (i) VH CDR1 consisting of a sequence of SEQ ID NO: 15, or a sequence having one or more amino acid substitutions, deletions, or additions (for example, 1, 2, or 3 amino acid substitutions, deletions, or additions) compared thereto,
   (ii) VH CDR2 consisting of a sequence of SEQ ID NO: 16, or a sequence having one or more amino acid substitutions, deletions, or additions (for example, 1, 2, or 3 amino acid substitutions, deletions, or additions) compared thereto, and
   (iii) VH CDR3 consisting of a sequence of SEQ ID NO: 17, or a sequence having one or more amino acid substitutions, deletions, or additions (for example, 1, 2, or 3 amino acid substitutions, deletions, or additions) compared thereto;
      and/or
(b) a light chain variable region (VL) comprising the following three complementary determining regions (CDR):
   (iv) VL CDR1 consisting of a sequence of SEQ ID NO: 18, or a sequence having one or more amino acid substitutions, deletions, or additions (for example, 1, 2, or 3 amino acid substitutions, deletions, or additions) compared thereto,
   (v) VL CDR2 consisting of a sequence of SEQ ID NO: 19, or a sequence having one or more amino acid substitutions, deletions, or additions (for example, 1, 2, or 3 amino acid substitutions, deletions, or additions) compared thereto, and
   (vi) VL CDR3 consisting of a sequence of SEQ ID NO: 20, or a sequence having one or more amino acid substitutions, deletions, or additions (for example, 1, 2, or 3 amino acid substitutions, deletions, or additions) compared thereto;

preferably, the substitution described in any one of (i)-(vi) is a conservative substitution; and
preferably, the VH of the antibody or the antigen binding fragment thereof comprises VH CDR1 as shown in SEQ ID NO: 15, VH CDR2 as shown in SEQ ID NO: 16, and VH CDR3 as shown in SEQ ID NO: 17; and the VL of the antibody or the antigen binding fragment thereof comprises: VL CDR1 as shown in SEQ ID NO: 18, VL CDR2 as shown in SEQ ID NO: 19, and VL CDR3 as shown in SEQ ID NO: 20.

On the one hand, the present invention provides an antibody or an antigen binding fragment thereof capable of specifically binding to an AXL protein, comprising:
(a) a heavy chain variable region (VH) comprising the following three complementary determining regions (CDR):
   (i) VH CDR1 consisting of a sequence of SEQ ID NO: 21, or a sequence having one or more amino acid substitutions, deletions, or additions (for example, 1, 2, or 3 amino acid substitutions, deletions, or additions) compared thereto,
   (ii) VH CDR2 consisting of a sequence of SEQ ID NO: 22, or a sequence having one or more amino acid substitutions, deletions, or additions (for example, 1, 2, or 3 amino acid substitutions, deletions, or additions) compared thereto, and
   (iii) VH CDR3 consisting of a sequence of SEQ ID NO: 23, or a sequence having one or more amino acid substitutions, deletions, or additions (for example, 1, 2, or 3 amino acid substitutions, deletions, or additions) compared thereto;
      and/or
(b) a light chain variable region (VL) comprising the following three complementary determining regions (CDR):
   (iv) VL CDR1 consisting of a sequence of SEQ ID NO: 24, or a sequence having one or more amino acid substitutions, deletions, or additions (for example, 1, 2, or 3 amino acid substitutions, deletions, or additions) compared thereto,
   (v) VL CDR2 consisting of a sequence of SEQ ID NO: 25, or a sequence having one or more amino acid substitutions, deletions, or additions (for example, 1, 2, or 3 amino acid substitutions, deletions, or additions) compared thereto, and
   (vi) VL CDR3 consisting of a sequence of SEQ ID NO: 26, or a sequence having one or more amino acid substitutions, deletions, or additions (for example, 1, 2, or 3 amino acid substitutions, deletions, or additions) compared thereto;

preferably, the substitution described in any one of (i)-(vi) is a conservative substitution; and
preferably, the VH of the antibody or the antigen binding fragment thereof comprises VH CDR1 as shown in SEQ ID NO: 21, VH CDR2 as shown in SEQ ID NO: 22, and VH CDR3 as shown in SEQ ID NO: 23; and the VL of the antibody or the antigen binding fragment thereof comprises: VL CDR1 as shown in SEQ ID NO: 24, VL CDR2 as shown in SEQ ID NO: 25, and VL CDR3 as shown in SEQ ID NO: 26.

On the one hand, the present invention provides an antibody or an antigen binding fragment thereof capable of specifically binding to an AXL protein, comprising:
(a) a heavy chain variable region (VH) comprising the following three complementary determining regions (CDR):
   (i) VH CDR1 consisting of a sequence of SEQ ID NO: 27, or a sequence having one or more amino acid substitutions, deletions, or additions (for example, 1, 2, or 3 amino acid substitutions, deletions, or additions) compared thereto,
   (ii) VH CDR2 consisting of a sequence of SEQ ID NO: 28, or a sequence having one or more amino acid substitutions, deletions, or additions (for example, 1, 2, or 3 amino acid substitutions, deletions, or additions) compared thereto, and
   (iii) VH CDR3 consisting of a sequence of SEQ ID NO: 29, or a sequence having one or more amino acid substitutions, deletions, or additions (for example, 1, 2, or 3 amino acid substitutions, deletions, or additions) compared thereto;
      and/or
(b) a light chain variable region (VL) comprising the following three complementary determining regions (CDR):
   (iv) VL CDR1 consisting of a sequence of SEQ ID NO: 30, or a sequence having one or more amino acid substitutions, deletions, or additions (for example, 1, 2, or 3 amino acid substitutions, deletions, or additions) compared thereto,
   (v) VL CDR2 consisting of a sequence of SEQ ID NO: 31, or a sequence having one or more amino acid substitutions, deletions, or additions (for example, 1, 2, or 3 amino acid substitutions, deletions, or additions) compared thereto, and
   (vi) VL CDR3 consisting of a sequence of SEQ ID NO: 32, or a sequence having one or more amino acid substitutions, deletions, or additions (for example, 1, 2, or 3 amino acid substitutions, deletions, or additions) compared thereto;

preferably, the substitution described in any one of (i)-(vi) is a conservative substitution; and
preferably, the VH of the antibody or the antigen binding fragment thereof comprises: VH CDR1 as shown in SEQ ID NO: 27, VH CDR2 as shown in SEQ ID NO: 28, and VH CDR3 as shown in SEQ ID NO: 29; and the VL of the antibody or the antigen binding fragment thereof comprises: VL CDR1 as shown in SEQ ID NO: 30, VL CDR2 as shown in SEQ ID NO: 31, and VL CDR3 as shown in SEQ ID NO: 32.

On the one hand, the present invention provides an antibody or an antigen binding fragment thereof capable of specifically binding to an AXL protein, comprising a heavy chain variable region and a light chain variable region, wherein
the heavy chain variable region comprises three CDRs contained in the heavy chain variable region shown in any one of SEQ ID NOs: 1, 3, 5, and 7; and the light chain variable region comprises three CDRs contained in the light chain variable region shown in any one of SEQ ID NOs: 2, 4, 6, and 8; and
preferably, the three CDRs contained in the heavy chain variable region and/or the three CDRs contained in the light chain variable region are defined by the Kabat, Chothia, or IMGT numbering system.

The antibody or the antigen binding fragment thereof of the present invention, wherein the antibody or the antigen binding fragment thereof comprises:
(a) a heavy chain variable region (VH) comprising an amino acid sequence selected from the group consisting of:
   (i) a sequence as shown in SEQ ID NO: 1;
   (ii) a sequence having one or more amino acid substitutions, deletions, or additions (e.g. 1, 2, 3, 4, or 5 amino acid substitutions, deletions, or additions) compared to the sequence as shown in SEQ ID NO: 1; or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 1;
      and/or,
(b) a light chain variable region (VL) comprising an amino acid sequence selected from the group consisting of:
   (iv) a sequence as shown in SEQ ID NO: 2;
   (v) a sequence having one or more amino acid substitutions, deletions, or additions (e.g. 1, 2, 3, 4, or 5 amino acid substitutions, deletions, or additions) compared to the sequence as shown in SEQ ID NO: 2; or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 2;

preferably, the substitution described in (ii) or (v) is a conservative substitution; and
preferably, the antibody or the antigen binding fragment thereof comprises: VH having the sequence as shown in SEQ ID NO: 1 and VL having the sequence as shown in SEQ ID NO: 2.

The antibody or the antigen binding fragment thereof of the present invention, wherein the antibody or the antigen binding fragment thereof comprises:
(a) a heavy chain variable region (VH) comprising an amino acid sequence selected from the group consisting of:
   (i) a sequence as shown in SEQ ID NO: 3;
   (ii) a sequence having one or more amino acid substitutions, deletions, or additions (e.g. 1, 2, 3, 4, or 5 amino acid substitutions, deletions, or additions) compared to the sequence as shown in SEQ ID NO: 3; or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 3;
      and/or,
(b) a light chain variable region (VL) comprising an amino acid sequence selected from the group consisting of:
   (iv) a sequence as shown in SEQ ID NO: 4;
   (v) a sequence having one or more amino acid substitutions, deletions, or additions (e.g. 1, 2, 3, 4, or 5 amino acid substitutions, deletions, or additions) compared to the sequence as shown in SEQ ID NO: 4; or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 4;

preferably, the substitution described in (ii) or (v) is a conservative substitution; and
preferably, the antibody or the antigen binding fragment thereof comprises: VH having the sequence as shown in SEQ ID NO: 3 and VL having the sequence as shown in SEQ ID NO: 4.

The antibody or the antigen binding fragment thereof of the present invention, wherein the antibody or the antigen binding fragment thereof comprises:
(a) a heavy chain variable region (VH) comprising an amino acid sequence selected from the group consisting of:
   (i) a sequence as shown in SEQ ID NO: 5;
   (ii) a sequence having one or more amino acid substitutions, deletions, or additions (e.g. 1, 2, 3, 4, or 5 amino acid substitutions, deletions, or additions) compared to the sequence as shown in SEQ ID NO: 5; or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 5;
      and/or,
(b) a light chain variable region (VL) comprising an amino acid sequence selected from the group consisting of:
   (iv) a sequence as shown in SEQ ID NO: 6;
   (v) a sequence having one or more amino acid substitutions, deletions, or additions (e.g. 1, 2, 3, 4, or 5 amino acid substitutions, deletions, or additions) compared to the sequence as shown in SEQ ID NO: 6; or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 6;

preferably, the substitution described in (ii) or (v) is a conservative substitution; and
preferably, the antibody or the antigen binding fragment thereof comprises: VH having the sequence as shown in SEQ ID NO: 5 and VL having the sequence as shown in SEQ ID NO:6.

The antibody or the antigen binding fragment thereof of the present invention, wherein the antibody or the antigen binding fragment thereof comprises:
(a) a heavy chain variable region (VH) comprising an amino acid sequence selected from the group consisting of:
   (i) a sequence as shown in SEQ ID NO: 7;
   (ii) a sequence having one or more amino acid substitutions, deletions, or additions (e.g. 1, 2, 3, 4, or 5 amino acid substitutions, deletions, or additions) compared to the sequence as shown in SEQ ID NO: 7; or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 7;
      and/or,
(b) a light chain variable region (VL) comprising an amino acid sequence selected from the group consisting of:
   (iv) a sequence as shown in SEQ ID NO: 8;
   (v) a sequence having one or more amino acid substitutions, deletions, or additions (e.g. 1, 2, 3, 4, or 5 amino acid substitutions, deletions, or additions) compared to the sequence as shown in SEQ ID NO: 8; or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 8;

preferably, the substitution described in (ii) or (v) is a conservative substitution; and
preferably, the antibody or the antigen binding fragment thereof comprises: VH having the sequence as shown in SEQ ID NO: 7 and VL having the sequence as shown in SEQ ID NO: 8.

The antibody or the antigen binding fragment thereof of the present invention, wherein the antibody or the antigen binding fragment thereof further comprises:
(a) a heavy chain constant region (CH) of a human immunoglobulin or a variant thereof having one or more amino acid substitutions, deletions, or additions (e.g. up to 20, up to 15, up to 10, or up to 5 amino acid substitutions, deletions, or additions, such as 1, 2, 3, 4 or 5 amino acid substitutions, deletions, or additions) as compared to the sequence from which it is derived; and
(b) a light chain constant region (CL) of a human immunoglobulin or a variant thereof having up to 20 amino acid conservative substitutions (e.g. up to 15, up to 10, or up to 5 amino acid conservative substitutions, such as 1, 2, 3, 4 or 5 amino acid conservative substitutions) as compared to the sequence from which it is derived;

preferably, the heavy chain constant region is an IgG heavy chain constant region, such as an IgG1, IgG2, IgG3, or IgG4 heavy chain constant region, more preferably, a human IgG1 or human IgG4 heavy chain constant region;
preferably, the light chain constant region is a kappa light chain constant region.

The antibody or the antigen binding fragment thereof of the present invention, wherein the antigen binding fragment is selected from the group consisting of Fab, Fab', (Fab') ₂, Fv, disulfide-linked Fv, scFv, diabody, and sdAb; and/or the antibody is a murine antibody, a chimeric antibody, a humanized antibody, a bispecific antibody or a multi-specific antibody; more preferably, the antibody is a fully human antibody.

On the other hand, the present invention provides a chimeric antigen receptor T cell comprising the antibody or the antigen binding fragment thereof of the present invention;
preferably, the heavy chain variable region and the light chain variable region in the antibody or the antigen binding fragment are combined in tandem or in parallel.

On the other hand, the present invention provides an isolated nucleic acid molecule that encodes the antibody or the antigen binding fragment thereof, or the heavy chain variable region and/or the light chain variable region thereof.

In another aspect, the present invention provides a vector comprising the isolated nucleic acid molecule of the present invention; preferably, the vector is a cloning vector or an expression vector; more preferably, the vector is a virus;

further preferably, the viral backbone of the viral vector is derived from a modified or engineered vaccinia virus Tiantan strain, vaccinia virus New York strain, vaccinia virus Copenhagen strain, vaccinia virus canarypox strain, vaccinia virus Ankara strain, adenovirus vector, adeno-associated virus vector, herpes simplex virus vector, varicella-zoster virus vector, respiratory syncytial virus, Semliki forest virus, Epstein-Barr virus, cytomegalovirus, human herpes virus type 6, variola virus, molluscum contagiosum virus, contagious ecthyma virus, respiratory enteric orphan virus, rotavirus, enterovirus, seneca virus, poliovirus, coxsackie virus, rhinovirus, hepatitis A virus, foot and mouth disease virus, togavirus, alphavirus, eastern equine encephalitis virus, sindbis virus, rubella virus, coronavirus, flavivirus, hepatitis C virus, Japanese encephalitis virus, st. Louis encephalitis virus, murray valley fever virus, yellow fever virus, west nile virus, zika virus, dengue virus, ebola virus, marburg virus, arenavirus, lassa fever virus, lymphocytic choriomeningitis virus, picinde virus, Junin virus, Machupo virus, Hantaan virus, rift Valley fever virus, paramyxovirus, human parainfluenza virus, mumps virus, simian virus 5, measles virus, vesicular stomatitis virus, rabies virus, respiratory syncytial virus, orthomyxovirus, influenza A virus, influenza B virus, influenza C virus, hepatitis D virus, simian immunodeficiency virus, human immunodeficiency virus type 1, human immunodeficiency virus type 2, rous sarcoma virus, human T-cell lymphotropic virus type-1, simian foamy virus, hepatitis B virus, hepatitis E virus, human papilloma virus, or polyoma virus.

More preferably, the vector is the cloning vector AbVec-hIgKappa (GenBank: FJ475056.1) or the cloning vector AbVec-hIgG1 (GenBank: FJ475055.1).

On the other hand, the present invention provides a host cell comprising the isolated nucleic acid molecule or the vector of the present invention;
preferably, the host cell is prokaryotic or eukaryotic, more preferably, the host cell is selected from *Escherichia coli* cells, yeast cells, mammalian cells or other cells suitable for the production of antibodies or antigen binding fragments, multi-specific antibodies; further preferably, the host cell is a mammalian cell; further more preferably, the host cell is a human, murine, ovine, equine, canine or feline cell; most preferably, the host cell is a 293 cell or a CHO cell.

In a further aspect, the present invention provides a method for preparing the antibody or the antigen binding fragment thereof of the present invention, comprising culturing the host cell under conditions that allow expression of the antibody or the antigen binding fragment thereof according to the present invention, and recovering the antibody or the antigen binding fragment thereof from the cultured host cell culture.

On the other hand, the present invention provides a bispecific or multi-specific molecule that includes the antibody or the antigen binding fragment thereof of the present invention;
preferably, the bispecific or multi-specific molecule specifically binds to an AXL protein and additionally specifically binds to one or more other targets;
preferably, the bispecific or multi-specific molecule further comprises at least one molecule having a second binding specificity for a second target (e.g., a secondary antibody); and
preferably, the bispecific or multi-specific molecule further comprises other antibodies or antigen binding fragments that specifically bind to an epitope of the AXL protein.

On the other hand, the present invention provides an immunoconjugate comprising the antibody or the antigen binding fragment of the present invention and a therapeutic agent connected to the antibody or the antigen binding fragment thereof;
preferably, the therapeutic agent is selected from a cytotoxic agent;
preferably, the therapeutic agent is selected from an alkylating agent, a mitotic inhibitor, an antitumor antibiotic, an antimetabolite, a topoisomerase inhibitor, a tyrosine kinase inhibitor, a radionuclide agent, and any combination thereof; and
preferably, the immunoconjugate is an antibody-drug conjugate (ADC).

On the other hand, the present invention provides a pharmaceutical composition comprising the antibody or the antigen binding fragment thereof, the bispecific or multi-specific molecule or the immunoconjugate of the present invention, as well as a pharmaceutically acceptable carrier and/or an excipient;
preferably, the pharmaceutical composition further comprises an additional pharmaceutically active agent;
preferably, the additional pharmaceutically active agent is a drug having anti-tumor activity, e.g., an alkylating agent, a mitotic inhibitor, an antitumor antibiotic, an antimetabolite, a topoisomerase inhibitor, a tyrosine kinase inhibitor, a radionuclide agent, a radiosensitizer, an antiangiogenic agent, a cytokine, a molecularly targeted drug, an immune checkpoint inhibitor, or an oncolytic virus; and
preferably, the antibody or the antigen binding fragment thereof, the bispecific or multi-specific molecule or the immunoconjugate, and the additional pharmaceutically active agent are provided as separate components or as components of the same composition.

On the other hand, the present invention provides a kit comprising the antibody or the antigen binding fragment of the present invention;
preferably, the antibody or the antigen binding fragment thereof carries a detectable label, for example, an enzyme (e.g. horseradish peroxidase), a radionuclide, a fluorescent dye, a luminescent substance (e.g. a chemiluminescent substance), or a biotin;
preferably, the kit further comprises a secondary antibody that specifically recognizes the antibody or the antigen binding fragment thereof of the present invention;
preferably, the secondary antibody further comprises a detectable label, such as an enzyme (e.g. horseradish peroxidase), a radionuclide, a fluorescent dye, a luminescent material (e.g. a chemiluminescent material), or a biotin.

On the other hand, the present invention provides a chimeric antigen receptor comprising an antigen binding domain of the antibody or the antigen binding fragment thereof of the present invention;
preferably, the antigen binding domain comprises a heavy chain variable region and a light chain variable region of the antibody or the antigen binding fragment thereof of the present invention;
preferably, the antigen binding domain is scFv;
preferably, the antigen binding receptor comprises an antigen binding fragment of the antibody of the present invention;
preferably, the antigen binding receptor is expressed by an immune effector cell (e.g., a T cell).

On the other hand, the present invention provides an isolated nucleic acid molecule that encodes the chimeric antigen receptor.

On yet another aspect, the present invention provides a vector comprising the isolated nucleic acid molecule encoding the chimeric antigen receptor; preferably, it being used to prepare the chimeric antigen receptor T cell.

On the other hand, the present invention provides a host cell comprising the isolated nucleic acid molecule or the vector encoding the chimeric antigen receptor;
preferably, the host cell is an immune effector cell (e.g. a T cell or an NK cell);
preferably, the host cell is a chimeric antigen receptor T (CAR-T) cell.

The present invention also provides a method for inhibiting the growth of a tumor cell and/or killing a tumor cell comprising contacting the tumor cell with an effective amount of the antibody or the antigen binding fragment thereof, the bispecific or multi-specific molecule, the immunoconjugate, the pharmaceutical composition, the chimeric antigen receptor, or the host cell as described herein.

The present invention also provides a method for preventing and/or treating a tumor in a subject (e.g. a human), the method comprising administering to the subject in need thereof an effective amount of the antibody or the antigen binding fragment thereof, the bispecific or multi-specific molecule, the immunoconjugate, the pharmaceutical composition, the chimeric antigen receptor, or the host cell as described herein;
preferably, the tumor is selected from B-cell lymphoma, T-cell lymphoma, melanoma, prostate cancer, renal cell carcinoma, sarcoma, glioma, such as high-grade glioma, blastoma, such as neuroblastoma, osteosarcoma, plasmacytoma, histiocytoma, pancreatic cancer, breast cancer, lung cancer, such as small cell lung cancer and non-small cell lung cancer, gastric cancer, liver cancer, colon cancer, rectal cancer, esophageal cancer, large intestine cancer, hematopoietic system cancer, testicular cancer, cervical cancer, ovarian cancer, bladder cancer, squamous cell cancer, adenocarcinoma, AIDS-related lymphoma, bladder cancer, brain cancer, cancer of the nervous system, head and neck cancer, squamous cell carcinoma of the head and neck, Hodgkin's lymphoma, non-Hodgkin' s lymphoma, or blood-borne neoplastic disease;
preferably, the subject is a mammal, such as a human;
preferably, the method further comprises administering an additional agent having anti-tumor activity, e.g., an alkylating agent, a mitotic inhibitor, an antitumor antibiotic, an antimetabolite, a topoisomerase inhibitor, a tyrosine kinase inhibitor, a radionuclide, a radiosensitizer, an antiangiogenic agent, a cytokine, a molecularly targeted drug, an immune checkpoint inhibitor, or an oncolytic virus;
preferably, the method further comprises administering an additional anti-tumor therapy, such as surgery, chemotherapy, radiation therapy, targeted therapy, immunotherapy, hormonal therapy, gene therapy, or palliative therapy.

The present invention also provides use of the antibody or the antigen binding fragment thereof, the bispecific or multi-specific molecule, the immunoconjugate, the pharmaceutical composition, the chimeric antigen receptor, or the host cell as described herein, in the manufacture of a medicament for the prevention and/or treatment of a tumor in a subject (e.g. a human);
preferably, the medicament further comprises an additional pharmaceutically active agent;
preferably, the additional pharmaceutically active agent is an agent having anti-tumor activity, e.g., an alkylating agent, a mitotic inhibitor, an antitumor antibiotic, an antimetabolite, a topoisomerase inhibitor, a tyrosine kinase inhibitor, a radionuclide agent, a radiosensitizer, an antiangiogenic agent, a cytokine, a molecularly targeted drug, an immune checkpoint inhibitor, or an oncolytic virus; and
preferably, the tumor is selected from B-cell lymphoma, T-cell lymphoma, melanoma, prostate cancer, renal cell carcinoma, sarcoma, glioma, such as high-grade glioma, blastoma, such as neuroblastoma, osteosarcoma, plasmacytoma, histiocytoma, pancreatic cancer, breast cancer, lung cancer, such as small cell lung cancer and non-small cell lung cancer, gastric cancer, liver cancer, colon cancer, rectal cancer, esophageal cancer, large intestine cancer, hematopoietic system cancer, testicular cancer, cervical cancer, ovarian cancer, bladder cancer, squamous cell cancer, adenocarcinoma, AIDS-related lymphoma, bladder cancer, brain cancer, cancer of the nervous system, head and neck cancer, squamous cell carcinoma of the head and neck, Hodgkin's lymphoma, non-Hodgkin' s lymphoma, or blood-borne neoplastic disease;
preferably, the subject is a mammal, such as a human.

Specifically, the method for preparing the antibody targeting AXL protein or the antigen binding fragment thereof provided by the present invention includes the steps of:
1) screening a natural phage antibody library to obtain a hybridoma cell strain capable of expressing an antibody targeting AXL protein or an antigen binding fragment thereof;
2) cloning a gene that expresses the antibody targeting an AXL protein or the antigen binding fragment thereof in the hybridoma cell strain obtained in step 1);
3) providing an expression vector comprising the cloned gene of step 2) and an expression control sequence operably linked to the gene;
4) transforming host cells using the expression vector of step 3);
5) culturing the host cell obtained in step 4); and
6) isolating and purifying the monoclonal antibody.

In another aspect, the present invention provides a hybridoma cell strain for use in the above preparation method.

In the present invention, 71 AXL-targeting antibodies are screened using the natural phage antibody library, all of which can recognize the human antibody binding to human AXL, and four AXL-targeting antibodies (Hu001-5, Hu001-7, Hu001-11, and Hu001-14) can specifically recognize the extra-cellular domain (ECD) protein of human AXL with the potential to block AXL-Gas6 interaction. The above-mentioned AXL antibodies have a stronger affinity under acidic conditions, which is expected to exert a more precise and effective tumor-killing effect and reduce toxic killing to normal tissues, thus increasing the safety of the clinical application of antibody drugs. The antibody targeting AXL of the present invention, which is capable of mediating the ADCC effect, can be used either as an individual drug antibody or in the preparation of CART cells to exert an anti-tumor effect.

In the present invention, a human-mouse hybridoma secreting an antibody specifically targeting AXL protein is prepared; the heavy and light chain sequences (100% of human genes) of the antibody are cloned using molecular biology techniques; and a human monoclonal antibody against AXL protein is constructed, expressed, and produced in CHO cells. These antibodies have greater binding capacity and specificity as drugs compared to existing antibodies. The experimental results show that the specific antibody of the present invention has a good biological effect, can recognize human AXL protein, with an affinity being 2.92 × 10⁻⁹ M and 1.40 × 10⁻⁹ M. The antibody of the present invention has strong chemotaxis in an acidic environment, can prepare therapeutic drugs such as CART or monoclonal antibodies for targeting diseases with high expression of AXL, and has a very good prospect.

Those skilled in the art will appreciate that the present invention described herein is susceptible to variations and modifications other than those specifically described. The present invention includes all these changes and modifications. The present invention also includes all of the steps and features mentioned or indicated in the specification, individually or collectively, and any and all combinations of steps or features.

By considering the following detailed description and drawings, other features and aspects of the present invention will become apparent.

### Brief Description of the Drawings

Hereinafter, embodiments of the present invention will be described in detail with reference to the accompanying drawings, wherein:
FIG. 1 shows a graph of the binding of clone supernatant to the antigen hAXL-ECD by ELISA screening. FIGs. 1A-1G respectively show that 71 clones of the present application can specifically bind to hAXL-ECD; clones 5, 7, 11, and 14 had the highest affinity, and clones 5, 7, 11, and 14 were selected and named Hu001-5, Hu001-7, Hu001-11, and Hu001-14.
FIG. 2 shows expression vector maps, AbVec-hIgKappa and AbVec-hIgG1 WT constructed for the antibody used in the present invention.

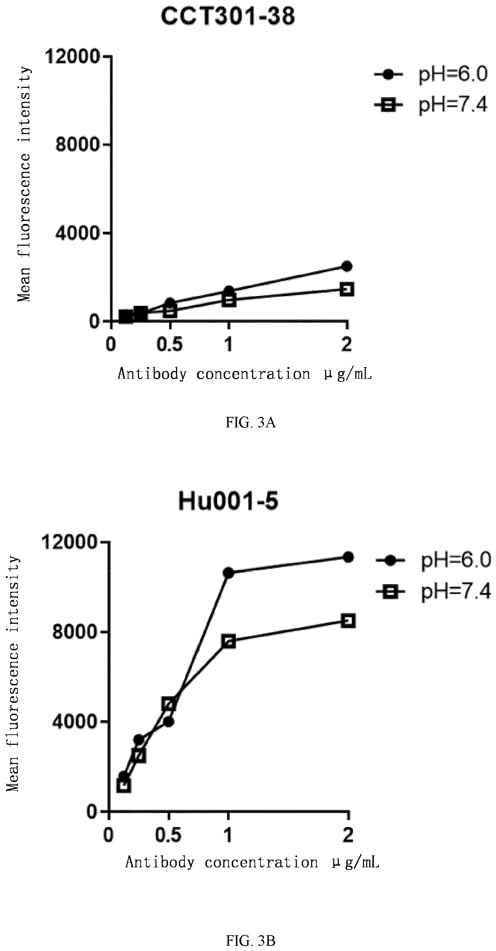

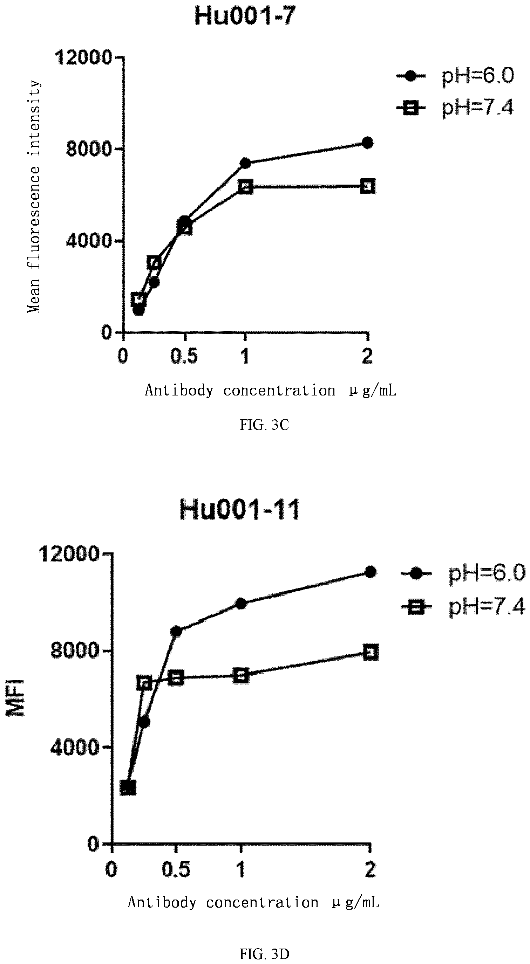

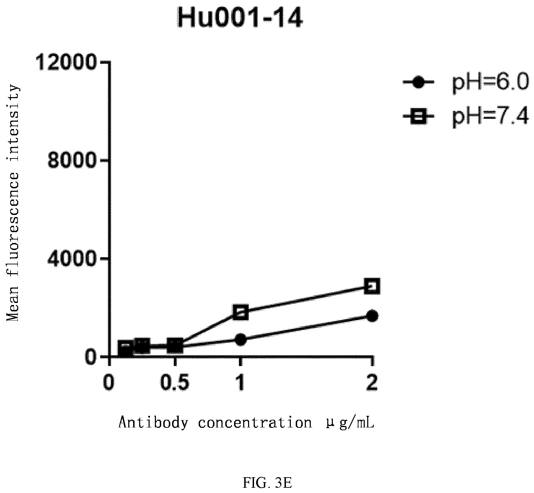

FIG. 3 is a graph of flow data showing the affinity of anti-AXL antibodies Hu001-5, Hu001-7, Hu001-11, and Hu001-14 of the present invention; where FIG. 3A shows a graphical representation of the affinity of the positive control CCT301-38 antibody as a function of concentration at different pH, and FIGs. 3B-3E show graphical representations of the affinity of the anti-AXL antibodies Hu001-5, Hu001-7, Hu001-11, and Hu001-14 as a function of concentration at different pH. It is apparent that the antibodies of the present invention have a higher affinity relative to the positive control and a higher binding capacity under acidic conditions than under neutral conditions.
FIG. 4 shows CART cells respectively prepared using the anti-AXL antibody Hu001-11 of the present invention and the positive control CCT301-38 antibody, and the *in vitro* activity tests under different conditions. It can be unambiguously seen from FIG. 4 that the anti-AXL antibody Hu001-11 of the present invention is more activated under acidic conditions *in vitro.*
FIG. 5 shows that CART cells prepared using the anti-AXL antibody Hu001-11 of the present invention can mediate higher killing activity *in vitro* compared to CART cells prepared using the positive control CCT301-38 antibody.
FIG. 6 shows that the anti-AXL antibodies Hu001-5, Hu001-7, Hu001-11, and Hu001-14 of the present invention can mediate a broader spectrum of anti-tumor activity *in vitro* as compared to the positive control CCT301-38 antibody.
FIG. 7 shows the arrangement of antibody variable regions.

### Mode of Carrying Out the Invention

The following description of the present application is merely illustrative of various embodiments of the present application. Therefore, the specific modifications discussed herein should not be construed as limiting the scope of the present application. Numerous equivalents, changes, and modifications will readily occur to those skilled in the art without departing from the scope of the present application, and it is understood that such equivalent embodiments are to be included within the scope of the present invention. All documents cited in the present application, including publications, patents, and patent applications, are incorporated herein by reference in their entirety.

### DEFINITION

The term "antibody" in the present invention includes any immunoglobulin, monoclonal antibody, polyclonal antibody, multi-specific antibody, or bispecific (diabody) antibody that binds to a particular antigen. A natural intact antibody comprises two heavy chains and two light chains. Each heavy chain is composed of a variable region and first, second, and third constant regions; each light chain consists of a variable region and a constant region. Mammalian heavy chains can be classified as alpha, delta, epsilon, gamma, and mu, and mammalian light chains as lambda or kappa. The antibody is "Y" shaped, with the neck of the Y structure consisting of the second and third constant regions of two heavy chains, which are joined by disulfide bonds. Each arm of the "Y"-shaped structure includes a variable region and a first constant region of one of the heavy chains in combination with the variable and constant regions of one light chain. The variable regions of light and heavy chains determine antigen binding. The variable region of each chain contains three highly variable regions, known as complementary determining regions (CDRs). The CDR of light chain (L) contains VLCDR1, VLCDR2, VLCDR3, and the CDR of heavy chain (H) contains VHCDR1, VHCDR2, VHCDR3. The CDR borders of the antibody and the antigen binding fragment disclosed in the present invention can be named or identified by Kabat, Chothia, or Al-Lazikani nomenclature. (AI-Lazikani, B., Chothia , C., Lesk, A.M., J. Mol. Biol., 273(4): 927(1997); Chothia, C. et. al, J. Mol. Biol., 186(3): 651-63(1985); Chothia, C. and Lesk, A. M., J. Mol. Biol., 196: 901 (1987); Chothia, C. et. al, Nature, 342(6252): 877-83 (1989); Kabat, E. A. et. al, National Institutes of Health, Bethesda, Md. (1991)). Among them, the three CDRs are separated by a lateral continuous portion called the frame region (FR), which is more highly conservative than CDR and forms a scaffold-supported hypervariable loop. The constant regions of the heavy and light chains are not involved in antigen binding, but have multiple effector functions. Antibodies can be divided into several classes based on the amino acid sequence of the heavy chain constant region. Depending on whether they contain alpha, delta, epsilon, gamma, and mu heavy chains, antibodies can be classified into five major classes or isomers, respectively: IgA, IgD, IgE, IgG, and IgM. Several major classes of antibodies can also be classified into subclasses, such as IgG1 (gamma 1 heavy chain), IgG2 (gamma 2 heavy chain), IgG3 (gamma 3 heavy chain), IgG4 (gamma 4 heavy chain), IgA1 (alpha 1 heavy chain), or IgA2 (alpha 2 heavy chain), etc.

The term "antigen binding fragment", as used herein, refers to an antibody fragment formed from an antibody comprising one or more CDR or any other antibody fragment that binds antigen but does not have the complete antibody structure. Examples of antigen binding fragments include, but are not limited to, antibodies such as diabody, Fab, Fab', F(ab')2, Fv fragments, disulfide stabilized Fv fragments (dsFv), (dsFv)2, bispecific dsFv (dsFv-dsFv'), disulfide stabilized diabody (ds diabody), single-chain antibody molecules (scFv), scFv dimer (bivalent bifunctional antibody), bivalent single chain antibody (BsFv), multi-specific antibody, camelized single domain antibody, nanobody, domain antibody, and bivalent domain antibody. Antigen binding fragments can bind to the same antigen as the maternal antibody. In certain embodiments, the antigen binding fragment can comprise one or more CDRs from a particular human antibody grafted to framework regions from one or more different human antibodies.

The Fab fragment of an antibody refers to the portion of an antibody molecule that is bound by disulfide bonds between a light chain (including a variable region and a constant region) and the variable and partially constant regions of a heavy chain.

A "Fab" fragment refers to a Fab segment that contains a portion of the hinge region.

"F(ab')2" refers to a dimer of Fab.

The Fc fragment of an antibody is responsible for many different effector functions such as ADCC and CDC, but is not involved in antigen binding.

The "Fv" fragment of an antibody refers to the smallest antibody fragment containing a complete antigen binding site. The Fv fragment consists of a light chain variable region and a heavy chain variable region.

A "single chain Fv antibody" or "scFv" refers to an engineered antibody that consists of a light chain variable region linked directly to a heavy chain variable region or linked through a peptide chain (Huston JS et al, Proc Natl Acad Sci USA, 85:5879(1988)).

A "single chain antibody Fv-Fc" or "scFv-Fc" refers to an engineered antibody consisting of scFv linked to the Fc portion of an antibody.

A "camelized single domain antibody", "heavy chain antibody" or "HCAb (Heavy-chain-only antibody)" all refer to an antibody containing two VH domains but no light chain (Riechmann L. and Muyldermans S., J Immunol Methods. 231(1-2): 25-38 (1999); Muyldermans S., J Biotechnol. 74(4): 277-302 (2001); WO94/04678; WO94/25591; U.S. Patent No. 6,005,079). Heavy chain antibodies were originally derived from camels (camels, unimodal camels, and llamas). Although lacking light chains, camelized antibodies have confirmed full antigen binding function (Hamers Casterman C.et al, Nature 363(6428): 446-8(1993); Nguyen VK.et al, "Heavy-chain antibodies in Camelidae: a case of evolutionary innovation, Immunogenetics. 54 (1): 39-47 (2002); Nguyen VK.et al, Immunology. 109(1): 93-101(2003)). The variable region (VH domain) of the heavy chain antibody is the smallest known antigen binding unit produced by acquired immunity (Koch-Nolte F., et al. FASEB J. 21 (13): 3490-8. Epub(2007)).

A "nanobody" refers to an antibody fragment consisting of one VH domain from a heavy chain antibody and two constant regions, i.e., CH2 and CH3.

A "diabody" includes a small antibody fragment with two antigen binding sites, wherein the fragment comprises a VH domain and a VL domain linked together on the same polypeptide chain (see Holliger P., et al. Proc Natl Acad Sci U S A. 90 (14): 6444-8(1993); EP404097; WO93/11161). The linker between the two domains is so short that the two domains on the same chain cannot pair with each other, thereby forcing the two domains to pair with complementary domains of the other chain to form two antibody binding sites. The two antibody binding sites may be targeted to bind the same or different antigens (or epitopes).

A "domain antibody" refers to an antibody fragment that contains only one heavy chain variable region or one light chain variable region. In some cases, two or more VH domains are covalently joined by a polypeptide linker and form a bivalent domain antibody. The two VH domains of a bivalent domain antibody can be targeted to the same or different antigens.

In certain embodiments, "(dsFv)2" comprises three peptide chains: the two VH genes are linked by a polypeptide linker and bound to two VL groups by disulfide bonds.

In certain embodiments, a "bispecific ds diabody" comprises a VL1-VH2 (linked by a polypeptide linker) and a VH1-VL2 (also linked by a polypeptide linker) that are disulfide-bonded between VH1 and VLl.

A "bispecific dsFv" or "dsFv-dsFv" contains three polypeptide chains: a VH1-VH2 group, wherein the heavy chains of both are linked by a polypeptide linker (e.g. a long elastic linker) and bound to the VL1 and VL2 groups, respectively, by disulfide bonds, each pair of heavy and light chains paired by disulfide bonds having a different antigen specificity.

In certain embodiments, a "scFv dimer" is a bivalent bifunctional antibody or bivalent single chain antibody (BsFv) comprising two VH-VL (linked by a polypeptide linker) groups that dimerize, wherein the VH of both groups cooperate with the VL of the other group to form two binding sites that can target the same antigen (or epitope) or different antigens (or epitopes). In other embodiments, a "scFv dimer" is a bispecific bifunctional antibody comprising V_{L1}-V_{H2} (linked by a polypeptide linker) and V_{H1}-V_{L2} (linked by a polypeptide linker) linked to each other, wherein V_{H1}, and V_{L1} cooperate, V_{H2} and V_{L2} cooperate, and each cooperating pair has a different antigen specificity.

The term "fully human" used in the present application, when used for an antibody or an antigen binding fragment, refers to the antibody or antigen binding fragment having a certain amino acid sequence or composed of the amino acid sequence, which corresponds to the amino acid sequence of antibody produced by humans or human immune cells, or derived from non-human sources such as genetically modified non-human animals using a human antibody library, or other sequences encoding human antibodies. In certain embodiments, a fully human antibody does not comprise amino acid residues (particularly antigen binding residues) derived from a non-human antibody.

As used herein, the term "humanized" when applied to an antibody or an antigen binding fragment refers to an antibody or an antigen binding fragment that includes a CDR derived from a non-human animal, an FR region derived from a human, and, where applicable, a constant region derived from a human. Because of their reduced immunogenicity, the humanized antibody or antigen binding fragment can be used as a therapeutic agent in humans in certain embodiments. In some embodiments, the non-human animal is a mammal such as a mouse, a rat, a rabbit, a goat, a sheep, a guinea pig, or a hamster. In some embodiments, the humanized antibody or antigen binding fragment consists essentially of all human sequences except that the CDR sequence is non-human. In some embodiments, the human-derived FR region may comprise the same amino acid sequence as the human-derived antibody from which it is derived, or it may comprise some amino acid e.g. no more than 10, 9, 8, 7, 6, 5, 4, 3, 2, or 1 amino acid change. In some embodiments, the amino acid change may be present only in the heavy chain FR region, only in the light chain FR region, or in both chains. In some preferred embodiments, the humanized antibody includes human FR1-3 and human JH and JK.

As used herein, the term "chimeric" refers to an antibody or an antigen binding fragment having a portion of a heavy and/or light chain derived from one species, and the remainder of the heavy and/or light chain derived from a different species. In one illustrative example, a chimeric antibody can include a constant region derived from a human and a variable region derived from a non-human animal such as a mouse.

As used herein, "specific binding" or "specifically binding" refers to a non-random binding reaction between two molecules, such as a reaction between an antibody and an antigen. In certain embodiments, an antibody or an antigen binding fragment thereof of the present application specifically binds to a human and/or a monkey AXL protein with a binding affinity (K_{D}) of ≤ 10⁻⁶ M. K_{D} in the present application refers to the ratio of dissociation rate to binding rate (k_{off}/kₒₙ) and can be determined by means of surface plasmon resonance, for example using an instrument such as Biacore.

Unless otherwise indicated, the heavy light chain constant regions of the antibody of the present invention are human IgG1 and kappa chains, respectively.

As used herein, "conservative substitution" when applied to an amino acid sequence refers to the replacement of one amino acid residue with another amino acid residue having a side chain with similar physicochemical properties. For example, conservative substitutions can be made between hydrophobic side chain amino acid residues (e.g. Met, Ala, VaL, Leu, and Ile), neutral hydrophilic side chain residues (e.g. Cys, Ser, Thr, Asn and Gln), acidic side chain residues (e.g. Asp and Glu), basic side chain amino acids (e.g. His, Lys, and Arg), or aromatic side chain residues (e.g. Trp, Tyr, and Phe). It is known in the art that conservative substitutions generally do not result in a significant change in the conformational structure of the protein, and thus retain the biological activity of the protein.

When "percent sequence identity" is used in reference to an amino acid sequence (or nucleic acid sequence), it refers to the percentage of amino acid (or nucleic acid) residues in a candidate sequence that are identical to the reference sequence as compared to the amino acid (or nucleic acid) residues in the candidate sequence after aligning the sequences and introducing gaps, if necessary, to maximize the number of identical amino acids (or nucleic acids). Conservative substitutions of the amino acid residues may or may not be considered to be identical residues. Sequences can be aligned by means disclosed in the art to determine the percent sequence identity of amino acid (or nucleic acid) sequences. A person skilled in the art can use the default parameters of the tool or adjust the parameters appropriately according to the needs of the alignment, for example by choosing a suitable algorithm.

As used herein, "T cells" include CD4+ T cells, CD8+ T cells, T helper type 1 T cells, T helper type 2 T cells, T helper type 17 T cells, and suppressor T cells.

As used herein, "effector function" refers to the biological activity of the Fc region of an antibody in binding to its effectors, such as a C1 complex and Fc receptor. Exemplary effector functions include complement-dependent CDC induced by the interaction of the antibody with C1q on the C1 complex, antibody-dependent cell-mediated cytotoxicity (ADCC) induced by binding of the Fc region of the antibody to the Fc receptor on the effector cell, and phagocytosis.

As used herein, "cancer" or "cancerous condition" refers to any medical condition that is mediated by the growth, proliferation, or metastasis of neoplastic or malignant cells and that causes solid tumors and non-solid tumors, such as leukemias. As used herein, a "tumor" refers to a solid mass of tumor and/or malignant cells.

A "treatment" or "therapy" of a condition includes preventing or alleviating the condition, reducing the rate of onset or development of the condition, reducing the risk of developing the condition, preventing or delaying the development of symptoms associated with the condition, reducing or stopping symptoms associated with the condition, causing a complete or partial reversal of the condition, curing the condition, or a combination thereof. The "treatment" or "therapy" with respect to cancer may refer to inhibiting or slowing the growth, proliferation, or metastasis of the tumor or malignant cell, or some combination thereof. The "treatment" or "therapy" of a tumor includes clearing all or a portion of the tumor, inhibiting or slowing tumor growth and metastasis, preventing or delaying tumor progression, or some combination thereof.

The "isolated" material has been artificially altered from its natural state. If a substance or component appears to be "isolated" in nature, it has been altered or removed from its original state, or both. For example, the naturally occurring polynucleotides or peptides in a living animal are not isolated, but if these polynucleotides or peptides coexist with a substance in their natural state sufficiently isolated and exist in a sufficiently pure state, they can be considered "isolated". In certain embodiments, the antibody and the antigen binding fragment have a purity of at least 90%, 93%, 95%, 96%, 97%, 98%, or 99%, as determined by electrophoretic methods (e.g. SDS-PAGE, isoelectric focusing, and capillary electrophoresis), or chromatographic methods (e.g. ion exchange chromatography or reverse phase HPLC).

As used herein, a "vector" refers to a vehicle into which a polynucleotide encoding a protein can be operably inserted to allow expression of the protein. The vector may be used to transform, transduce, or transfect a host cell so that the elements of genetic material it carries are expressed in the host cell. For example, a vector includes a plasmid, phagemid, cosmid, artificial chromosomes such as yeast artificial chromosomes (YAC), bacterial artificial chromosomes (BAC) or P1-derived artificial chromosomes (PAC), phages such as lambda phage or M13 phage, and animal virus, etc. Among the types of animal viruses used as vectors include a retrovirus (including a lentivirus, adenovirus, adeno-associated virus, herpes virus (e.g. herpes simplex virus), pox virus, baculovirus, papillomavirus, and papovavirus (e.g. SV40)). The vector may contain a variety of elements for controlling expression, including a promoter sequence, a transcription initiation sequence, an enhancer sequence, a selection element, and a reporter gene. In addition, the vector may contain an origin of replication. The vector may also include components that facilitate its entry into the cell, including, but not limited to, a viral particle, a liposome, or a protein shell.

As used herein, a "host cell" is a cell into which an exogenous polynucleotide and/or a vector has been introduced.

### With regard to the antibody targeting AXL protein in the present invention

In certain embodiments, the present application provides exemplary AXL protein-targeted antibodies Hu001-5 antibody, Hu001-7 antibody, Hu001-11 antibody, and Hu001-14 antibody.

It will be appreciated by those skilled in the art that the aforementioned CDR sequences may be modified to include a substitution of one or more amino acids, thereby resulting in increased biological activity such as increased binding affinity to an AXL protein. For example, phage display technology can be used to produce and express libraries of antibody variants (e.g. Fab or FcFv variants), followed by screening for antibodies that have affinity to the AXL protein. In another example, computer software can be used to model the binding of the antibody to the AXL protein and identify amino acid residues on the antibody that form a binding interface. Substitution of these residues may be avoided to prevent a decrease in binding affinity or may be targeted for substitution to form a stronger binding. In certain embodiments, at least one (or all) of the substitutions in the CDR sequence is a conservative substitution.

In certain embodiments, the antibody or the antigen binding fragment includes one or more CDR sequences that have at least 80% (e.g. at least 85%, 88%, 90%, 91%, 92%, 93%, 94%, 95%, 96%, 97%, 98%, and 99%) sequence identity to the sequence of SEQ ID NOs: 9-32 while retaining similar or even higher binding affinity to the AXL protein than its parent antibody. The parent antibody has essentially the same sequence, but its corresponding CDR sequence has 100% sequence identity to the sequence set forth in SEQ ID NOs: 9-32.

In some embodiments, the antibody or the antigen binding fragment described herein is capable of specific binding to an AXL protein with a binding affinity (KD) of ≤ 10⁻⁷M, as measured by surface plasmon resonance. Binding affinity values can be expressed as the K_{D} value, which is calculated as the ratio of the dissociation rate to the binding rate (k_{off}/kₒₙ) when the binding of antigen and antigen binding molecule reaches equilibrium. The antigen binding affinity (e.g. K_{D}) may suitably be determined by suitable methods known in the art, including, for example, plasmon resonance binding using an instrument such as Biacore.

In certain embodiments, the antibody or the antigen binding fragment described herein binds to the AXL protein with an EC50 (i.e. median effective concentration) of 1 ng/mL - 10 µg/mL. The binding of the antibody or the antigen binding fragment to the AXL protein can be determined by methods known in the art such as sandwich methods such as ELISA, Western blotting, FACS, or other binding assays. In an illustrative example, the antibody to be tested (i.e. the primary antibody) is bound to an immobilized AXL protein or cells expressing the AXL protein, followed by washing away unbound antibody, and a labeled secondary antibody is introduced, which is capable of binding to the primary antibody so that bound secondary antibody can be detected. The detection can be performed on a microplate reader plate when using the immobilized AXL protein, or using FACS analysis when using cells expressing AXL protein.

In certain embodiments, the antibody or the antigen binding fragment described herein binds to an AXL protein with an EC50 (i.e. 50% effective concentration) of 10 ng/mL-10 µg/mL (as determined using the FACS assay).

In some embodiments, the antibody described herein are advantageous in that they can be used in combination with immunogenic substances, such as tumor cells, purified tumor antigens and cells transfected with immuno-stimulators, and tumor vaccines. In addition, such antibodies and the antigen binding fragment thereof that target the AXL protein can be included in combination therapies, including standard chemotherapy and radiotherapy, target-based small molecule therapies, and other emerging immune checkpoint modulator therapies. In some embodiments, the antibodies and antigen binding fragments thereof can be used as base molecules for antibody-drug conjugates, bispecific or multivalent antibodies.

In some embodiments, the antibody and the antigen binding fragment thereof described herein are camelized single chain domain antibody, bifunctional antibody (diabody), scFv, scFv dimer, BsFv, dsFv, (dsFv)2, dsFv-dsFv', Fv fragment, Fab, Fab', F(ab')2, ds diabody, nanobody, domain antibody, or bivalent domain antibody.

In some embodiments, the antibody described herein comprises an immunoglobulin constant region. In some embodiments, the immunoglobulin constant region comprises a heavy chain and/or a light chain constant region. The heavy chain constant region comprises a CH1, CH1-CH2 or CH1-CH3 region. In some embodiments, the immunoglobulin constant region may further comprise one or more modifications to achieve desired properties. For example, the constant region may be modified to reduce or eliminate one or more effector functions to enhance FcRn receptor binding or to introduce one or more cysteine residues.

In some embodiments, the antibody and its antigen binding fragment further comprise a conjugate. It is contemplated that the antibody or the antigen binding fragment thereof of the present invention may be linked to a variety of conjugates (see, e.g. "Conjugate Vaccines", Contributions to Microbiology and Immunology, J. M. Cruse and R. E. Lewis, Jr. (eds.), Carger Press, New York(1989)). These conjugates may be linked to the antibody or the antigen conjugate by covalent binding, affinity binding, intercalating, coordinate binding, complexing, binding, mixing, or adding, among other means. In certain embodiments, the antibodies and the antigen binding fragments disclosed herein can be engineered to contain specific sites other than epitope-binding moieties that can be used to bind one or more conjugates. For example, such sites may comprise one or more reactive amino acid residues, such as cysteine residues and histidine residues, for facilitating covalent attachment to the conjugate. In certain embodiments, the antibody can be indirectly linked to the conjugate, or linked through another conjugate. For example, the antibody or the antigen binding fragment thereof can bind to biotin and then indirectly to a second conjugate that is linked to avidin. The conjugate can be a detectable label, a pharmacokinetic modifying moiety, a purification moiety, or a cytotoxic moiety. Examples of detectable labels may include a fluorescent label (e.g. fluorescein, rhodamine, dansyl, phycoerythrin, or Texas red), an enzyme substrate label (e.g. horseradish peroxidase, alkaline phosphatase, luciferase, glucoamylase, lysozyme, carbohydrate oxidase, or beta-D-galactosimase), a stable isotope or a radioisotope, a chromophore moiety, a digoxin, a biotin/avidin, a DNA molecule, or gold for detection. In certain embodiments, the conjugate may be a pharmacokinetic modifying moiety such as PEG, which helps to extend the half-life of the antibody. Other suitable polymers include, for example, carboxymethyl cellulose, dextran, polyvinyl alcohol, polyvinylpyrrolidone, ethylene glycol/propylene glycol copolymer, and the like. In certain embodiments, the conjugate can be a purification moiety such as a magnetic bead. A "cytotoxic moiety" can be any agent that is harmful to cells or that may damage or kill cells. Examples of cytotoxic moieties include but are not limited to, paclitaxel, cytochalasin B, gramicidin D, ethidium bromide, emetine, mitomycin, etoricoxib, teniposide, vincristine, vinblastine, colchicine, adriamycin, daunorubicin, dihydroxy anthracin dione, mitoxantrone, mithramycin, actinomycin D, 1-dehydrotestosterone, glucocorticoid, procaine, tetracaine, lidocaine, propranolol, puromycin and analogs thereof, antimetabolite (e.g. methotrexate, 6-mercaptopurine, 6-mercaptoguanine, cytarabine, 5-fluorouracil dacarbazine), alkylating agents (e.g. chlormethine, thiotepa chlorambucil, melphalan, carmustine (BSNU) and lomustine (CCNU), cyclophosphamide, busulfan, dibromomannitol, streptozotocin, mitomycin C and cis-diammin-odichloroplatinum (DDP) cisplatin), anthracycline (e.g. daunorubicin (formerly daunomycin) and adriamycin), antibiotic (e.g. dactinomycin (formerly actinomycin), bleomycin, mithramycin and anthramycin (AMC)) and antimitotic agent (e.g. vincristine and vinblastine).

### Polynucleotide and recombinant method

By using well-known genetic engineering techniques in this field, the amino acid sequences of the antibody and the antigen binding fragment thereof described in the present application can be converted into corresponding DNA coding sequences. Due to the degeneracy of the genetic code, the transformed DNA sequences can be identical while the encoded protein sequences remain unchanged.

The vector comprising the polynucleotide encoding the antibody and the antigen binding fragment thereof can be introduced into a host cell for DNA or gene expression using recombinant techniques known in the art. In another embodiment, the antibody and the antigen binding fragment thereof can be made by homologous recombination methods known in the art. A variety of vectors are available. Vector components typically include, but are not limited to, two or more of the following: signal sequences, the origin of replication, one or more marker genes, enhancer sequences, promoters (e.g.: SV40, CMV, EF-1a), and transcription termination sequences.

In some embodiments, the vector systems include mammalian, bacterial, yeast systems, and the like, and will include plasmids such as, but not limited to, pALTER, pBAD, pcDNA, pCal, pL, pELpGEMEX, pGEX, pCLpCMV, pEGFP, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS420, pLexA, pACT2, and other vectors available in the laboratory or on the market. Suitable vectors may include a plasmid or a viral vector (e.g. replication-defective retrovirus, adenovirus, and adeno-associated virus).

Vectors comprising polynucleotides encoding the antibody and the antigen binding fragment thereof can be introduced into the host cell for cloning or gene expression. The host cells suitable for cloning or expressing DNA in the vector of the present invention are prokaryotic cells, yeast, or higher eukaryotic cells as described above. Prokaryotic cells suitable for use in the present invention include eubacteria, such as Gram-negative or Gram-positive bacteria, for example, *enterobacteriaceae* (e.g. *E*. *coli), enterobacterium genus, Erwinia, Klebsiella, proteus, salmonella,* e.g. *Salmonella typhimurium (S. typhi),* SerratiaBizio, e.g. *Serratia marcescens,* and *Shigella,* and *Bacillus,* e.g. *Bacillus subtilis* and *Bacillus licheniformis, pseudomonas,* e.g. P. *aeruginosa,* and *Streptomyces.*

In addition to prokaryotic cells, eukaryotic microorganisms such as filamentous fungi or yeast can also be used as host cells to clone or express vectors encoding antibodies. Saccharomyces cerevisiae, or baker's yeast, is the most commonly used lower eukaryotic host microorganism. However, many other genera, species and strains are common and suitable for use in the present invention, such as Schizosaccharomyces pombe; Kluyveromyces hosts such as *Kluyveromyces lactis, kluyveromyces fragilis* (ATCC12424), *kluyveromyces bulgaricus* (ATCC16045), *kluyveromyces wickeramii* (ATCC24178), *kluyveromyces waltii* (ATCC56500), *kluyveromyces drosophilarum* (ATCC36906), *kluyveromyces thermotolerans* and *Kluyveromyces marxianus: Yarrowia lipolytica* (EP402226); *pichia pastoris* (EP183070); *candida: trichoderma reesei* (EP244234); *neurospora;* western Schwann yeasts such as: *western Schwann yeast;* and *filamentous fungi,* such as: neurospora, penicillium, tolypocladium and aspergillus, such as: *aspergillus nidulans* and *Aspergillus niger.*

Host cells suitable for expression of glycosylated antibody or the antigen binding fragment thereof provided herein are derived from multicellular organisms. Examples of invertebrate cells include plant and insect cells. A number of *baculoviral strains* and variants thereof and corresponding permissive insect host cells have been discovered, from hosts such as *Spodoptera frugiperda (J.E.Smmith)* (Lepidoptera), *Stegomyia αegyptz* (mosquitoe), *Aedes albopictus* (mosquitoe), *Drosophila melanogaster* (fruit fly) and *Bombyx mori Linnaeus.* A variety of viral strains are publicly available for transfection, such as *Autographa californica* nuclear polyhedrosis virus and Bm-5 variants of *Bombyx mori* nuclear polyhedrosis virus, all of which can be used in the present invention, particularly for transfection of *Spodoptera frugiperda* cells. Plant cell cultures of cotton, corn, potato, soybean, petunia, tomato, and tobacco can also be used as hosts.

However, spinal cells are of most interest, and culture (tissue culture) of spinal cells has become routine. Examples of useful mammalian host cells are the SV40 transformed monkey kidney cell CV1 line (COS-7, ATCC CRL 1651); human embryonic kidney cell lines (293 or suspension cultured 293 cell subclones, Graham et al.,]. Gen Virol.36: 59(1977)); baby hamster kidney cells (ATCC CCL 10); Chinese hamster ovary cells/-DHFR (CHO, Urlaub et al., Proc. Natl. Acad. Sci. USA 77:4216(1980)); mouse sertoli cells (TM4, Ma ther, Biol. Reprod. 23:243-251(1980)); monkey kidney cells (CV1ATCC CCL 70); African green monkey kidney cells (VERO-76, ATCC CRL-1587); human cervical cancer cells (HELA, ATCC CCL 2); canine kidney cells (MDCK, ATCC CCL 34); Buffalo rat hepatocytes (BRL 3A, ATCC CRL 1442); human lung cells (W138, ATCC CCL75); human hepatocytes (Hep G2, HB 8065); mouse mammary tumor (MMT 060562, ATCC CCL51); TRI cells (Mather et al. Annals N. Y Acad. Sci.383: 44-68(1982)); MRC 5 cells; FS4 cells; and human liver cancer cell line HepG2). In some preferred embodiments, the host cells are 293F cells.

Host cells are transformed with the expression or cloning vectors described above that produce the antibody and the antigen binding fragment thereof, and cultured in conventional nutrient media modified as appropriate for inducing the promoter, selecting transformed cells, or amplifying the gene encoding the sequence of interest.

Host cells used in the present invention to produce the antibody and the antigen binding fragment thereof can be cultured in a variety of media known in the art. The medium may also contain any other necessary additives at appropriate concentrations known in the art. The conditions of the medium, such as temperature, pH, and the like, are well known to those of ordinary skill in order to select previously used conditions for the host cell for expression.

Where recombinant techniques are used, the antibody may be produced intracellularly, in the wall membrane space, or secreted directly into the culture medium. If the antibody is produced intracellularly, particulate debris from host cells or lytic fragments is first removed, for example, by centrifugation or sonication. Carter et al., Bio/Technology 10: 163-167(1992) describe a method for isolating antibodies secreted into the wall membrane space of *E*. *coli.* Briefly, a cell paste can be dissolved in the presence of uranium acetate (pH 3.5), EDTA, and PMSF for more than about 30 minutes. The cell debris can be removed by centrifuge. If the antibody is secreted into the culture medium, the supernatant of the expression system is typically first concentrated using a commercially available protein concentration filter, such as lAmicon or Millipore Pellicon ultrafiltration unit. A protease inhibitor such as PMSF may be added in any of the foregoing steps to inhibit protein degradation, as well as antibiotics to prevent the growth of adventitious contaminants.

The antibody prepared from the cell can be purified using purification methods such as hydroxyapatite chromatography, gel electrophoresis, dialysis, DEAE-cellulose ion exchange chromatography columns, ammonium sulfate precipitation, salting-out, and affinity chromatography, with affinity chromatography being the preferred purification technique. The type of antibody and the Fc domain of any immunoglobulin present in the antibody determine whether protein A is suitable as an affinity ligand. Protein A can be used to purify antibodies based on human gamma 1, gamma 2, or gamma 4 heavy chains (Lindmark et al., J. Immunol. Meth. 62:13(1983)). Protein G applies to all murine isoforms and human gamma 3 (Guss et al., EMBO J.5: 1567 1575(1986)). Agarose is the most commonly used affinity ligand attachment matrix, but other substrates can also be used. Mechanically stable substrates such as controlled pore glass or poly (styrene) benzene allow faster flow rates and shorter processing times than agarose. If the antibody contains a CH3 domain, it can be purified using Bakerbond ABX. TM resin (J. T. Baker, Phillipsburg, N. J). Other techniques for protein purification, such as fractionation on an ion exchange column, ethanol precipitation, reverse phase HPLC, chromatography on silica gel, chromatography on heparin sepharose based on anion or cation exchange resins (such as a polyaspartic acid column), chromatofocusing, SDS-PAGE, and ammonium sulfate precipitation, may also be used depending on the antibody to be obtained.

After any preliminary purification step, the mixture containing the antibody of interest and impurities can be treated by low pH hydrophobic interaction chromatography, using an elution buffer at a pH of about 2.5-4.5, preferably at a low salt concentration (e.g. from about 0 to 0.25M salt concentration).

### Kit

The present application provides a kit comprising the antibody or the antigen binding fragment thereof. In some embodiments, the kit is used to detect the presence or level of AXL protein in a biological sample. The biological sample may comprise cells or tissue.

In some embodiments, the kit comprises an antibody or the antigen binding fragment thereof conjugated with a detectable marker. In some embodiments, the kit comprises an unlabeled antibody and further comprises a secondary antibody capable of binding to the unlabeled antibody. The kit may further comprise instructions for use and a packaging separating each component in the kit.

In some embodiments, the antibody is linked to a substrate or instrument for use in a sandwich assay such as an ELISA or immunochromatography assay. Suitable substrates or instruments can be, for example, microwell plates and test strips.

### Pharmaceutical composition and therapeutic method

The present application further provides a pharmaceutical composition comprising the antibody and one or more pharmaceutically acceptable carriers.

Pharmaceutically acceptable carriers used in the pharmaceutical compositions disclosed herein may include, for example, a pharmaceutically acceptable liquid, gel, or solid carrier, an aqueous media, a non-aqueous media, an antimicrobial material, an isotonic material, a buffer, an antioxidant, an anesthetic, a suspending/dispersing agent, an integrating agent, a diluent, an adjuvant, an adjuvants, or a non-toxic auxiliary substance, and other ingredients well known in the art, or combinations of the above.

Suitable components may include, for example, an antioxidant, a filler, a binder, a disintegrant, a buffer, a preservative, a lubricant, a flavoring agent, a thickener, a coloring agent, an emulsifier, or a stabilizer such as a sugar and a cyclodextrin. Suitable antioxidants may include, for example, methionine, ascorbic acid, EDTA, sodium thiosulfate, platinum, catalase, citric acid, cysteine, thioglycerol, thioglycolic acid, thiosorbitol, butyl methyl anisole, butylated hydroxytoluene, and/or propyl gallate. The inclusion of one or more antioxidants, such as methionine, in a composition comprising the antibody disclosed herein will reduce the oxidation of the antibody. A reduction in oxidation prevents or reduces a decrease in binding affinity, thereby improving antibody stability and extending shelf life.

Further, the pharmaceutically acceptable carrier can include, for example, aqueous media such as sodium chloride injection, Ringer's injection, isotonic dextrose injection, sterile water injection, or dextrose, and lactated Ringer's injection, non-aqueous media such as: fixed oil of vegetable origin, cottonseed oil, corn oil, sesame oil, or peanut oil, antibacterial substance in bacteriostatic or fungistatic concentration, isotonic agent such as sodium chloride, or glucose, buffer such as phosphate, or citrate buffer, antioxidant such as sodium bisulfate, local anesthetic such as procaine hydrochloride, suspending aid and dispersing agent such as sodium carboxymethyl cellulose, hydroxypropylmethyl cellulose or polyvinylpyrrolidone, emulsifier such as polysorbate 80 (Tween-80), integrating reagent such as EDTA (ethylenediamine tetraacetic acid) or EGTA (ethylene glycol bis (2-aminoethyl ether) tetraacetic acid), ethanol, polyethylene glycol, propylene glycol, sodium hydroxide, hydrochloric acid, citric acid, or lactic acid. An antimicrobial agent as a carrier may be added to the pharmaceutical composition in a multi-dose container, including phenol or cresol, mercurial, benzyl alcohol, chlorobutanol, methyl and propyl p-hydroxybenzoate, thimerosal, Chlorophenylmethane ammonium, and chlorophenethyl ammonium. Suitable adjuvants may include, for example, water, saline, dextrose, glycerol, or ethanol. Suitable non-toxic auxiliary substances may include, for example, emulsifiers, pH buffers, stabilizers, solubilizers, or substances such as sodium acetate, sorbitan laurate, triethanolamine oleate, or cyclodextrins.

The pharmaceutical composition may be a liquid solution, a suspension, an emulsion, a pill, a capsule, a tablet, a sustained-release formulation, or a powder. Oral formulations can include standard carriers such as pharmaceutical-grade mannitol, lactose, starch, magnesium stearate, polyvinylpyrrolidone, sodium saccharin, cellulose, magnesium carbonate, and the like.

In certain embodiments, the pharmaceutical composition is formulated into an injectable composition. The injectable pharmaceutical composition may be prepared in any conventional form, for example, a liquid solvent, a suspending agent, an emulsifying agent or a solid form suitable for the production of the liquid solvent, the suspending agent, or the emulsifying agent. The formulation for injection may include sterile and/or pyrogen-free solutions ready for use, a sterile dry soluble, such as lyophilized powders, sterile suspension agent including subcutaneous tablets, sterile suspensions ready for injection, sterile dry insoluble products that are currently combined with the medium before use, and sterile and/or pyrogen-free emulsion. The solvent may be aqueous or non-aqueous.

In certain embodiments, a unit dose of an injectable preparation is packaged in an ampoule, a vial, or a syringe with a needle. It is known in the art that all preparations for injection should be sterile and pyrogen-free.

In certain embodiments, sterile lyophilized powders can be prepared by dissolving the antibody or the antigen binding fragment thereof disclosed herein in an appropriate solvent. The solvent may contain an additional pharmacological component that improves the stability of the powder or reconstituted solution prepared from the powder or improves the powder or reconstituted solution. Suitable adjuvants include but are not limited to, water, glucose, sorbitol, fructose, corn syrup, xylitol, glycerol, glucose, black sugar, or other suitable substances. The solvent may contain a buffer, such as a citrate buffer, a sodium or potassium phosphate buffer, or other buffers known to those skilled in the art. In an embodiment, the pH of the buffer is neutral. Subsequent sterile filtration of the solution followed by lyophilization under standard conditions well known in the art produces the desired formulation. In an embodiment, the resulting solvent is dispensed into vials for lyophilization. Each vial may contain a single dose or multiple doses of the antibody or the antigen binding fragment thereof or composition thereof that targets the AXL protein. The loading in each vial may be slightly higher than that required for each dose or for multiple doses (e.g. a 10% excess), thereby ensuring accurate sampling and dosing. The lyophilized powder can be stored under appropriate conditions, such as in the range of about 4°C to room temperature.

The freeze-dried powder is re-dissolved with injection water to obtain a formulation for injection administration. In an embodiment, the lyophilized powder can be re-dissolved by adding it to sterile pyrogen-free water or another suitable liquid carrier. The precise amount is determined by the choice of therapy and may be determined empirically.

Also provided is a treatment method comprising administering a therapeutically effective amount of the antibody described herein to a subject in need thereof.

The therapeutically effective dosage of the antibody provided herein depends on a variety of factors well known in the art, such as weight, age, past medical history, current therapy, the subject's health status and potential for cross-infection, allergies, hypersensitivity, and side effects, as well as the route of administration and extent of tumor progression. One skilled in the art (e.g. a physician or veterinarian) can proportionally reduce or increase the dosage according to these or other conditions or requirements.

In certain embodiments, the antibody provided herein can be administered at a therapeutically effective dose of between about 0.01 mg/kg to about 100 mg/kg. In certain embodiments, the antibody is administered at a dose of about 50 mg/kg or less, in certain embodiments 10 mg/kg or less, 5 mg/kg or less, 1 mg/kg or less, 0.5 mg/kg or less, or 0.1 mg/kg or less. A particular dose may be administered at intervals such as once daily, twice or more daily, twice or more monthly, once weekly, once every two weeks, once every three weeks, once monthly, or once every two or more months. In certain embodiments, the dosage administered may vary over the course of treatment. For example, in certain embodiments, the initial dose may be higher than the subsequent dose. In certain embodiments, the dosage administered is adjusted over the course of treatment according to the response of the subject to whom it is administered.

The dosage regimen may be adjusted to achieve the optimal response (e.g. a therapeutic response). For example, a single dose may be administered or multiple divided doses may be administered over a period of time.

The antibodies disclosed herein can be administered by any means known in the art, such as by injection (e.g. subcutaneous, intraperitoneal, intravenous, including intravenous drip, intramuscular, or intradermal) or by non-injection (e.g. oral, nasal, sublingual, rectal, or topical).

In certain embodiments, the antibodies can be used to treat disorders related to their molecular mechanism, including tumors and cancers, such as those selected from B-cell lymphoma, T-cell lymphoma, melanoma, prostate cancer, renal cell carcinoma, sarcoma, glioma, such as high-grade glioma, blastoma, such as neuroblastoma, osteosarcoma, plasmacytoma, histiocytoma, pancreatic cancer, breast cancer, lung cancer, such as small cell lung cancer and non-small cell lung cancer, gastric cancer, liver cancer, colon cancer, rectal cancer, esophageal cancer, large intestine cancer, hematopoietic system cancer, testicular cancer, cervical cancer, ovarian cancer, bladder cancer, squamous cell cancer, adenocarcinoma, AIDS-related lymphoma, bladder cancer, brain cancer, cancer of the nervous system, head and neck cancer, squamous cell carcinoma of the head and neck, Hodgkin's lymphoma, non-Hodgkin' s lymphoma, or blood-borne neoplastic disease.

### Method of Use

The present application further provides a method for using the antibody.

In some embodiments, provided herein is a method for treating a condition or disorder associated with the antibody mechanism in an individual comprising administering a therapeutically effective amount of the antibody described herein.

The antibody disclosed herein can be administered alone or in combination with one or more other therapeutic means or substances. For example, the antibody disclosed herein can be used in combination with chemotherapy, radiation therapy, cancer treatment surgery (e.g. tumor resection), an antiviral drug, treatment with one or more anti-emetics or other chemotherapy-induced complications, or any other therapeutic agent for cancer or virus. In certain such embodiments, the antibody disclosed herein, when used in combination with one or more therapeutic substances, may be administered simultaneously with the one or more therapeutic substances, and in certain such embodiments, the antibody may be administered simultaneously as part of the same pharmaceutical composition. However, an antibody "in combination with" another therapeutic substance need not be administered simultaneously or in the same composition as the therapeutic substance. The meaning of "in combination with" in the present invention also includes that an antibody that is administered before or after another therapeutic agent is also considered to be "in combination with" that therapeutic agent, even if the antibody is administered differently from the second agent. Where possible, the other therapeutic substances to be used in combination with the antibody disclosed in the present invention may be administered by reference to the methods described in the product specifications for the other therapeutic substances or by reference to the surgeon's Desk Reference 2003 (Physicians' Desk Reference, 57th Ed; Medical Economics Company; ISBN: 1563634457; 57th edition (November 2002)), or other methods known in the art.

In certain embodiments, the therapeutic substance is capable of inducing or enhancing an immune response against cancer. For example, tumor vaccines can be used to induce an immune response to certain tumors or cancers. Cytokine therapy can be used to enhance the presentation of tumor antigens to the immune system. Examples of cytokine therapy include but are not limited to, interferons such as interferons alpha, beta, and gamma, colony stimulating factors such as macrophage CSF, granulocyte macrophage CSF and granulocyte CSF, interleukins such as IL-L, IL-1a, IL-2, IL-3, IL-4, IL-5, IL-6, IL-7, IL-8, IL-9, IL-10, IL-11, and IL-12, tumor necrosis factors such as TNF-alpha, and TNF-beta. Agents that inactivate immunosuppressive targets may also be used, such as a PD-1 antibody, a TGF-beta inhibitor, an IL-10 inhibitor, and a Fas ligand inhibitor. Another group of agents includes those that activate immune responses against tumors or cancer cells, e.g. those that increase T cell activation (e.g. T cell costimulatory signaling pathways such as CTLA-4, ICOS, OX40, 4-1BB, etc. pathways), as well as those that increase dendritic cell function and antigen presentation.

The following examples are intended to better illustrate the present invention and should not be construed as limiting the scope thereof. All of the specific compositions, materials, and methods described below, in whole or in part, are within the scope of the present invention. These specific compositions, materials, and methods are not intended to limit the present invention but are merely illustrative of specific embodiments within the scope of the present invention. Those skilled in the art may develop equivalent compositions, materials, and methods without adding an inventive step and without departing from the scope of the present invention. It should be understood that various modifications in the methods of the present invention may still be included within the scope of the present invention. The inventors intend such variations to be included within the scope of the present invention.

### Example 1: Construction and screening of a natural human antibody phage display library

AXL Antigen Preparation: human recombinant AXL protein was purchased from novoprotein, cat. No: C02B.

The AXL fully human antibody screening was entrusted to Sanyou Biomedical (Shanghai) Co. Ltd. for completion. A total of four rounds of screening were performed and the clones obtained in the fourth round were selected for ELISA screening of positive clones for ELISA. Finally, a total of 71 positive clones able to bind to the hAXL-ECD protein were selected out of 2304 clones. After sequencing and ELISA binding, four clones were selected to construct full-length antibodies for further experiments. The specific implementation methods were as follows:

### 1.1 Positive clone sequencing and analysis

After primary screening, 71 positive clones that could bind to hAXL-ECD protein were numbered. 2 µL of bacterial solution was pipetted into 2 mL of 2YT medium, and cultured overnight at 37°C at 220 rpm. The plasmid was extracted for second-generation sequencing. Sequencing results processing: the original AB1 files were integrated by SeqMan and aligned. Non-antibody gene sequences were removed to generate a fasta file with the antibody gene being integrated. Subsequently, the DNA sequence was translated into an amino acid sequence through MEGA6. The amino acid sequences containing terminators, unconventional sequences, etc were found through the amino acid sequence. A fasta file of the amino acid sequence was exported to obtain the antibody light chain and heavy chain sequences of the positive antibody clone. After analysis and alignment, the sequences were completely new antibody sequences.

### 1.2 Affinity of clone supernatants for binding to antigen hAXL-ECD by ELISA screening

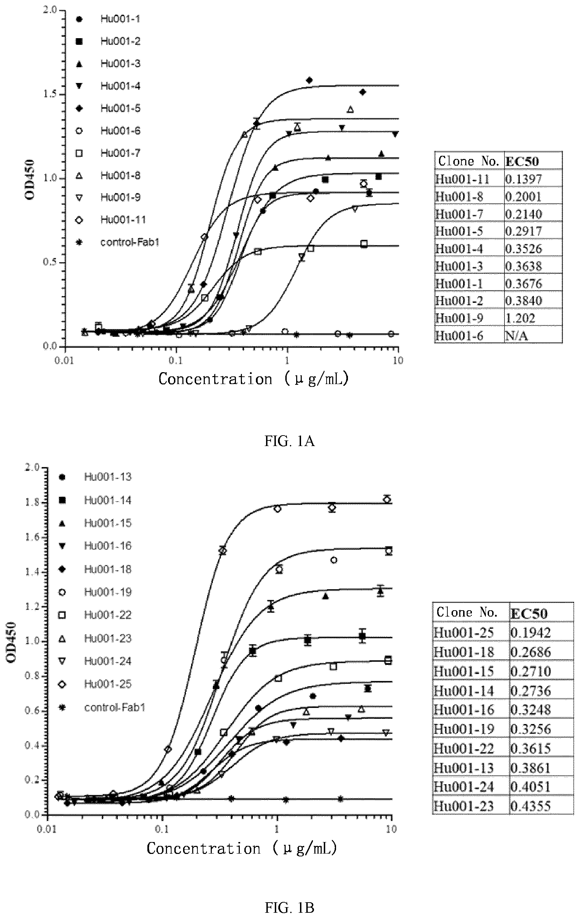

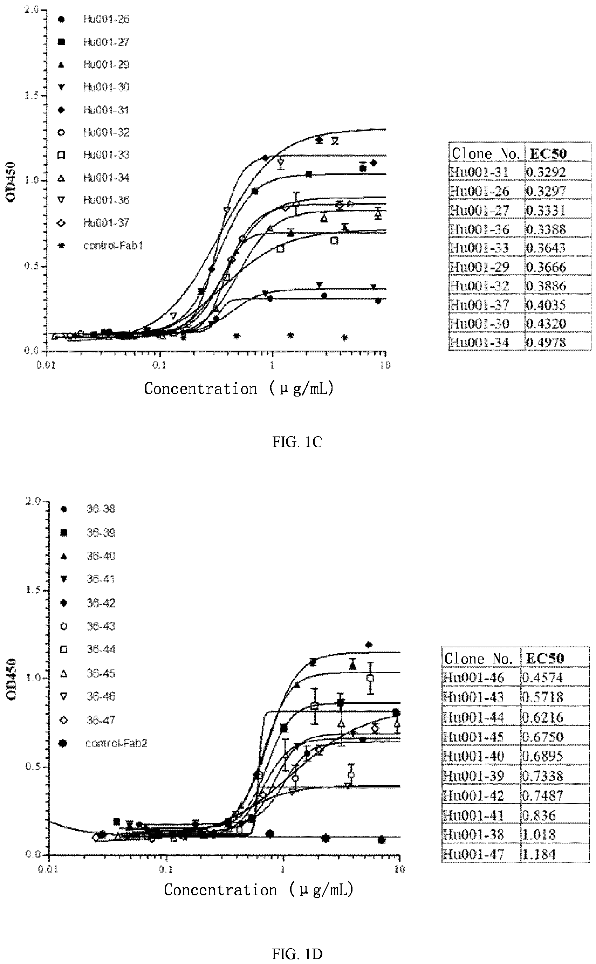

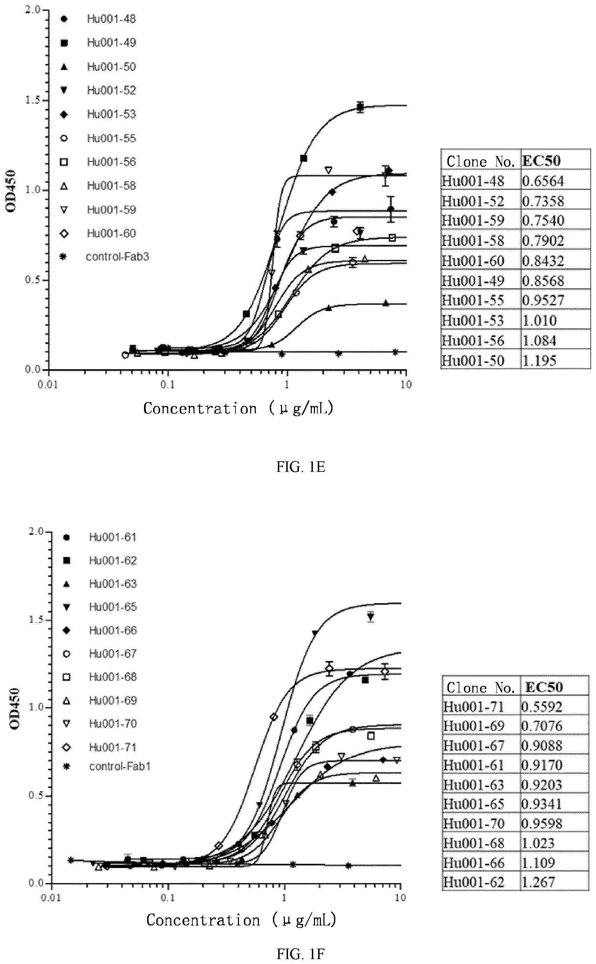

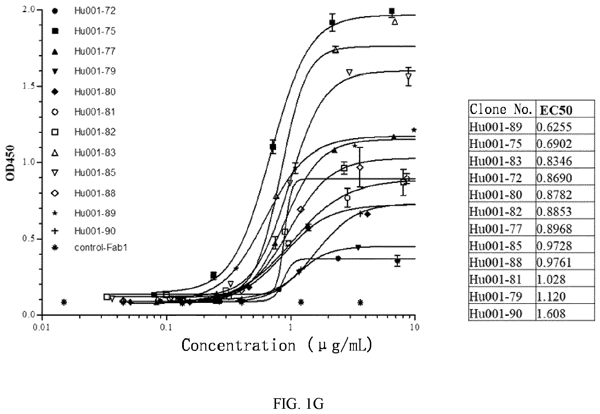

Clones from the third round of selection were first picked up in a 96-well deep-well plate containing 300 µL of 2-YT medium and incubated overnight at 37°C. The supernatant, which contained the expressed Fab, was taken, diluted in a gradient, and added to an ELISA plate coated with 2 µg/mL of hAXL-ECD. HRP-labeled goat anti-human Fab was used as the secondary antibody (goat anti-human-HRP, ThermoFisher, 31482, 1: 6000 dilution) for detection. A higher signal value indicated a stronger affinity. The results were shown in Figures 1A-G, which respectively showed that 71 clones of the present application can specifically bind to hAXL-ECD; clones 5, 7, 11, and 14 had the highest affinity, and clones 5, 7, 11, and 14 were selected and named Hu001-5, Hu001-7, Hu001-11, and Hu001-14. The Fab of four antibodies (Hu001-5 antibody, Hu001-7 antibody, Hu001-11 antibody, and Hu001-14 antibody) all showed good affinity activity in the ELISA assay.

The cDNA sequence of the heavy chain variable region and the cDNA sequence of the light chain variable region of the secreted antibody were obtained by sequencing. The corresponding antibody CDR sequences are shown in Table 1.

**Table 1 Antibodies and the CDR sequences of the present invention**

| **Antibody No.** | **Domain** | **Sequence** | |
|---|---|---|---|
| | | **Amino Acid** | **SEQ ID NO:** |
| Hu001-5 | H-CDR | HCDR1: SYGMH | 9 |
| | | HCDR2: VISYDGSNKYYADSVKG | 10 |
| | | HCDR3: DQVLAPVGGGLFDY | 11 |
| | L-CDR | LCDR1: RASQSISTYLN | 12 |
| | | LCDR2: AASSLAS | 13 |
| | | LCDR3: QQSYSTPRT | 14 |
| | VH | QVQLLESGGGVVQPGRSLRLSCAASGFTFSSYGM HWVRQAPGKGLEWVAVISYDGSNKYYADSVKGR FTISRDNSKNTLYLQMNSLRAEDTAVYYCAKDQV LAPVGGGLFDYWGQGTLVTVSAA | 1 |
| | VL | DIQLTQSPSSLSASVGDRVTITCRASQSISTYLNWY QQKPGKAPESLIFAASSLASGVPSRFSGSGSGTDFT LTISSLQPEDFATYYCQQSYSTPRTFGPGTKVEIK | 2 |
| Hu001-7 | H-CDR | HCDR1: SYAIS | 15 |
| | | HCDR2: GIIPIFGTANYAQKFQGR | 16 |
| | | HCDR3: DIVSRVRGELVGGYYYFGLDV | 17 |
| | L-CDR | LCDR1: QQSYSTPRT | 18 |
| | | LCDR2: AASRLQS | 19 |
| | | LCDR3: QQSYSTPRT | 20 |
| | VH | QVQLVQSGAEVKKPGSSVKVSCKASGGTFSSYAI SWVRQAPGQGLEWMGGIIPIFGTANYAQKFQGRV TITADESTSTAYMELSSLRSEDTAVYYCAKDIVSR VRGELVGGYYYFGLDVWGQGTTVTVSAA | 3 |
| | VL | NIQLTQSPSSLSASVGDRVTITCRASQTISSYLNWY QQKPGKAPKLLIYAASRLQSGVPSRFSGSGSGTDF TLTISSLQPEDFATYYCQQSYSTPRTFGQGTKVEIK | 4 |
| Hu001-11 | H-CDR | HCDR1: SYGMH | 21 |
| | | HCDR2: VISYDGSNKYYADSVKG | 22 |
| | | HCDR3: YGSSGPYWYFDL | 23 |
| | L-CDR | LCDR1: RASQSISSYLN | 24 |
| | | LCDR2: AASSLQS | 25 |
| | | LCDR3: QQSYSTPRT | 26 |
| | VH | QVQLLESGGGVVQPGRSLRLSCAASGFTFSSYGMH WVRQAPGKGLEWVAVISYDGSNKYYADSVKGRFTI SRDNSKNTLYLQMNSLRAEDTAVYYCAKYGSSGPY WYFDLWGQGTLVTVSAA | 5 |
| | VL | DIQLTQSPSSLSASVGDRVTITCRASQSISSYLNWYQ QKPGKAPKLLIYAASSLQSGVPSRFSGSGSGTDFTLT ISSLQPEDFATYYCQQSYSTPRTFGQGTKLEIR | 6 |
| Hu001-14 | H-CDR | HCDR1: SYYMH | 27 |
| | | HCDR2: IINPSGGSTSYAQKFQG | 28 |
| | | HCDR3: DWYFDV | 29 |
| | L-CDR | LCDR1: RASQGISSWLA | 30 |
| | | LCDR2: AASSLQS | 31 |
| | | LCDR3: QQANSFPF | 32 |
| | VH | EVQLVQSGAEVKKPGASVKVSCKASGYTFTSYYMH WVRQAPGQGLEWMGIINPSGGSTSYAQKFQGRVTM TRDTSTSTVYMELSSLRSEDSAVYYCARDWYFDVW GAGTTVTVSAA | 7 |
| | VL | NIQMTQSPSSVSASVGDRVTITCRASQGISSWLAWY QQKPGKAPKLLIYAASSLQSGVPSRFSGSGSGTDFTL TISSLQPEDFATYYCQQANSFPFTFGGGTKVEIK | 8 |

### Example 2 Construction, expression, and purification of full-length antibodies

In this example, the two Fab antibodies obtained in Example 1, which had good blocking activity for binding to hAXL-ECD, were constructed as human IgG1 subtypes, in which the light chain was of κ type and the antibody was fully human.

### 2.1 Construction of plasmid

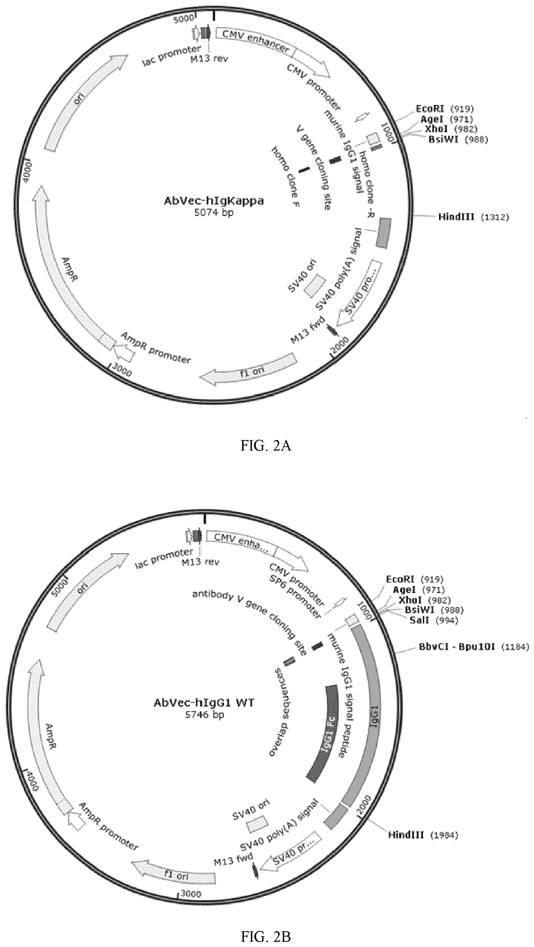

From the screened antibody-containing strains, the variable region fragments of antibody light and heavy chains were amplified by PCR and respectively constructed into eukaryotic expression vector plasmids AbVec-hIgKappa or AbVec-hIgG1 WT (FIG. 2) containing the constant region fragments of light and heavy chains by homologous recombination method to constitute the complete full-length genes of antibody light and heavy chains encoding the antibody heavy and light chains amino acid sequences as shown in SEQ ID NOs:1-8.

The constructed vectors containing full-length antibody light-heavy chain genes were separately transformed into *E*. *coli* TOP10 (WEIDI, DL1010S) and cultured overnight at 37°C. The antibody light and heavy chain plasmids were extracted by the endotoxin-free plasmid extraction kit (OMEGA, D6950-01) for eukaryotic expression.

### 2.2 Expression and purification of antibodies

The candidate antibodies Hu001-5, Hu001-11, Hu001-7, Hu001-14, and the control antibody CCT301-38 (see US10066238) were expressed by the Expi293 transient expression system (Thermo Fisher, cat. No. A1435101) as follows: on the day of transfection of Expi293 cells cultured in a 250 mL culture flask, the cell density was confirmed to be around 7 × 10⁶ viable cells/mL, as well as cell viability was> 98%. The cells were adjusted to a final concentration of 6 × 10⁶ cells/mL (final volume 75 mL) using an Expi293 expression medium pre-warmed at 37°C. 1 mL of Expi293^{™} expression medium precooled at 4°C was taken to dilute the target plasmid (50 µg). Meanwhile, 1 mL of Expi293 TM expression medium was used to dilute the transfection reagent 75 µL of FectoPro (Polyplus, cat. No. PT-116-001). Then the two were mixed in equal volume of 1 mL to prepare an Expi293^{™} expression medium/plasmid DNA mixture by gently mixing, which was incubated at room temperature for 15 min and slowly added to a prepared cell suspension. The mixture was placed in a cell culture shaker and cultured at 37°C, 5% CO₂. At 24 h after the transfection, FectoPRO booster was added to the culture (with a final concentration of 0.6 µL/mL) and incubation was continued at 37°C with shaking and 5% CO₂. On day 5 after the transfection, the same volume of Expi293^{™} expression medium was slowly added to the culture system. On day 10 after the transfection, the cell culture supernatant was collected and centrifuged at 4000 g for 10 min. After affinity purification on a Protein G agarose column (GE, cat. No. 28903134), the protein of interest was eluted with 100 mM sodium acetate (pH3.0). After neutralization with 1M Tris-HCl, the resulting protein was replaced into a PBS buffer by a 30 kD ultrafiltration concentration tube (Millipore, cat. No. UFC901096).

The four candidate antibodies Hu001-5, Hu001-7, Hu001-11, Hu001-14, and the control antibody CCT301-38 all have a relative molecular weight of 150kD and a purity of more than 90%. The protein concentration of the purified antibody was measured with an ultra-micro spectrophotometer (Thermo Fisher, model NanoDrop One C), and the value obtained by dividing the measured A280 value by the theoretical extinction coefficient of the antibody was taken as the antibody concentration value. The antibody was packaged and stored at -80°C.

### Example 3 Affinity testing of candidate antibodies for specific binding to hAXL-ECD

The affinity of the candidate antibody for specific binding to hAXL-ECD was detected by flow cytometry. A total of 4 clones of Hu001-5, Hu001-7, Hu001-11, and Hu001-14 were tested under acidic (pH = 6.0) and neutral (pH = 7.4) conditions, respectively.

CHO-AXL cells (constructed by Shanghai Sinobay Biotechnology Co. Ltd.) were cultured in a cell culture flask in DMEM/F-12 medium (10% FBS, 1% penicillin/streptomycin). After 48 hours of culture at 37°C in an incubator containing 5% CO₂, the supernatant was discarded. The cells were collected by digestion with 0.25% trypsin and centrifuged at 800 g for 3 minutes. The supernatant was discarded and the cells were resuspended (10⁶/mL) in 2% FBS Wash Buffer. The pH of the buffer was adjusted and the cells were divided into pH6.0 and pH7.4 groups, and primary antibodies (Hu001-5 antibody, Hu001-7 antibody, Hu001-11 antibody, Hu001-14 antibody, and positive control antibody CCT301-38) were added to each group and incubated for 15 minutes at room temperature. The supernatant was discarded. Fluorescent-labeled secondary antibody IgG-Fc-PE (BioLegend, cat. No. 409304) was added and incubation was continued at room temperature for 15 min in the dark. Then, the detection was performed on the machine. A higher signal value can indicate a stronger affinity.

The results were shown in FIG. 3, which was a graph of flow data showing the affinity of anti-AXL antibodies Hu001-5, Hu001-7, Hu001-11, and Hu001-14 of the present invention; where

FIG. 3A showed a graphical representation of the affinity of the positive control CCT301-38 antibody as a function of concentration at different pH, and FIGs. 3B-3E showed graphical representations of the affinity of the anti-AXL antibodies Hu001-5, Hu001-7, Hu001-11, and Hu001-14 as a function of concentration at different pH. It is apparent that the antibodies of the present invention have a higher affinity relative to the positive control and a higher binding capacity under acidic conditions than under neutral conditions. As can be seen from the graph, the mean fluorescence intensities of the Hu001-5 antibody experimental group and the Hu001-11 antibody experimental group were about 4 times that of the positive control group under both acidic and neutral conditions. The mean fluorescence intensity of Hu001-5 antibody experimental group and Hu001-11 antibody experimental group under an acidic condition was higher than that under a neutral condition, which was about 1.5 times the latter. It can be concluded that Hu001-5 and Hu001-11 both have higher affinity for AXL than the positive control and have higher binding capacity under the acidic condition than under the neutral condition.

### Example 4 In vitro activity assay of CART cells prepared with acid-sensitive antibody Hu001-11

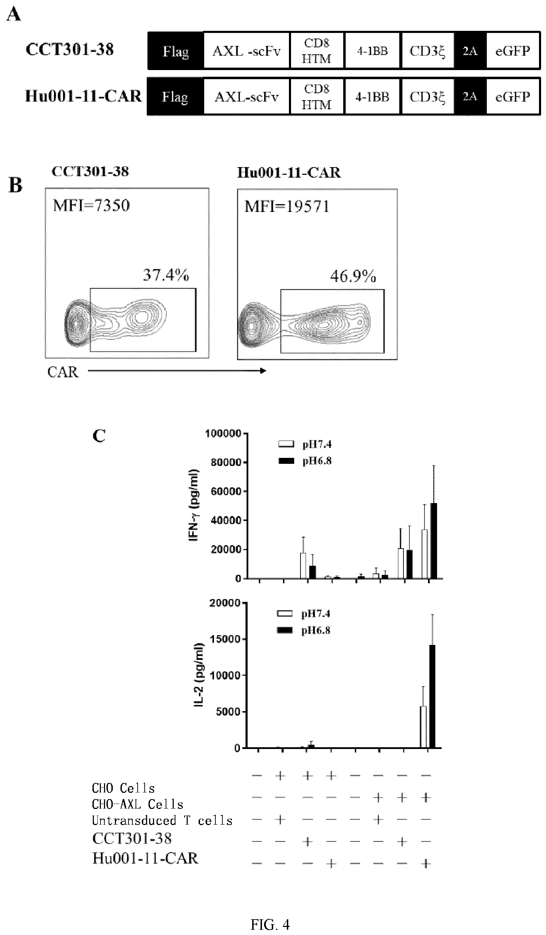

The structures of the CART cell chimeric antigen receptors for Hu001-11 and the positive control were shown in FIG. 4A.

Hu001-11-CART cells and positive control CCT301-38-CART cells were prepared by Shanghai Sinobay Biotechnology Co. Ltd. Flow cytometry was used to detect the positive rate of CAR expression and mean fluorescence intensity. As shown in FIG. 4B, the positive rate of control antibody CCT301-38 was 37.4%, and the mean fluorescence intensity (MFI) was 7350. The positive rate of Hu001-11-CAR cells was 46.9%, and the mean fluorescence intensity (MFI) was 19571. The *in vitro* transduction efficiency of Hu001-11-CART cells was much higher than that of control CART cells.

The CHO-AXL cells were added to a 96-well plate as target cells (10⁴ cells/well). The prepared Hu001-11-CART cells were effector cells, and the ratio of the effector cells to the target cells was 2: 1. Control effector cells included non-transduced T cells (negative control) and CCT301-38-CART cells (positive control). Cell culture was divided into a pH6.8 group and a pH7.4 group. After co-culture at 37°C in a 5% CO₂ incubator for 24 hours, the contents of IL-2 and IFN-γ were detected by ELISA method to determine the degree of *in vitro* activation of CART cells.

The protein concentrations of IL-2 (Abclonal, cat. No. RK00002) and IFN-γ (Abclonal, cat. No. RK00015) in the supernatant of cultured cells were determined by Elisa. The results were shown in FIGs. 4C and D. Hu001-11-CART cells secreted higher levels of IL-2 and IFN-γ than the positive control CCT301-38-CART cells in the CHO-AXL *in vitro* co-culture and secreted higher levels in the acidic condition. It was suggested that Hu001-11-CART cells were more strongly activated under the acidic condition *in vitro* and had good chemotaxis in an acidic environment.

### Example 5: In vitro glioma cell killing experiment mediated by Hu001-11 antibody

The target cells in this experiment were human glioma cells U251. The effector cells were CD16-CART cells (CD16-T cells, prepared by Shanghai Sinobay Biotechnology Co. Ltd.). The U251 cell line was cultured in DMEM medium (10% FBS, 1% penicillin/streptomycin) at 37°C in a 5% CO₂ incubator. The cells were plated in an 8-well culture chamber at a cell concentration of 10⁵/mL. After 12 hours, Hu001-11 antibody and CD16-T cells were added, respectively, at a ratio of the effector cells to the target cells of 2: 1. Untransduced T cells were added separately as a negative control, CCT301-38 antibody, and CD16-T cells as positive controls. Cell index, which correlates positively with the number of viable cells, was measured in real-time using the xCELLigence RTCA S16 instrument. A higher value can indicate a lower killing activity. Conversely, higher killing activity is indicated.

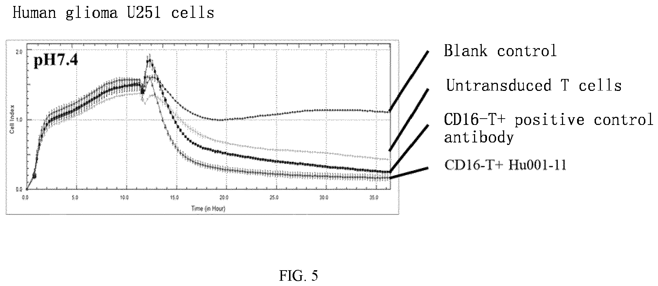

The results were shown in FIG. 5. The combination of Hu001-11 and CD16-T resulted in significant killing of U251 cells at 12 hours and almost complete killing of target cells at 20 hours. The positive controls showed complete killing at 24 hours. It was suggested that Hu001-11 mediated better killing activity on human glioma U251 cells.

### Example 6 In Vitro broad spectrum anti-tumor activity assay mediated by the antibody of the present invention

Also verified was that the anti-AXL antibodies Hu001-5, Hu001-7, Hu001-11, and Hu001-14 of the present invention mediate killing experiments of CD16-T cells *in vitro* against other solid tumor cells (human ovarian adenoma cell SK-OV3 and human lung carcinoma cell NCI-H292 tumors, all bearing a reporter gene encoding the luciferase).

Reagents and instruments used were purchased from Promega unless otherwise specified. The specific steps were as follows: Tumor cells were plated in a 96-well plate (10⁴/well). After 24 h of incubation at 37°C, 5% CO₂, the medium was removed and anti-AXL antibodies of the present invention, Hu001-5, Hu001-7, Hu001-11, and Hu001-14 were added. The cells were divided into ten groups: namely, positive control antibody CCT301-38 group, Hu001-5 antibody group, Hu001-7 antibody group, Hu001-11 antibody group, Hu001-14 antibody group, Hu001-5 antibody combined with CD16-T cell group, Hu001-7 antibody combined with CD16-T cell group, Hu001-11 antibody combined with CD16-T cell group, Hu001-14 antibody combined with CD16-T cell group, and CCT301-38 combined with CD16-T cell group. The ratio of CD16-T cells to target cells was 2: 1. After further incubation at 37°C for 24 h, the supernatant was removed and 50 µL of 1x cell lysate (Cat. No. E1531) was added to each well. After 30 min of incubation at room temperature with shaking, 30 µL of luciferase assay substrate (cat. No. E151A) was added to each well and developed for 30 s. The fluorescence value was detected on the machine (GloMax^{®} Navigator Microplate Luminometer).

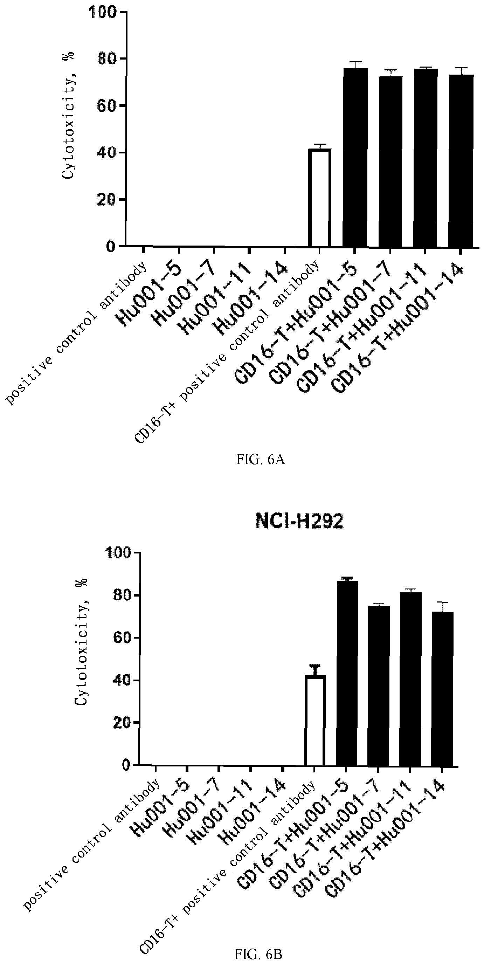

The results were shown in FIG. 6. The anti-AXL antibodies, Hu001-5, Hu001-7, Hu001-11, and Hu001-14 of the present invention, mediated the killing of both tumor cell lines *in vitro* more than 1.5-fold more efficiently than the positive control antibody, CCT301-38, with significant killing enhancement, indicating that the anti-AXL antibodies of the present invention can mediate a broader spectrum of anti-tumor activity *in vitro.*

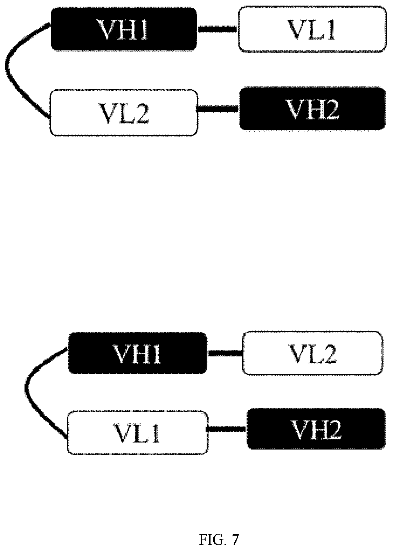

FIG. 7 showed the arrangement of antibody variable regions.

While particular embodiments of the present invention have been illustrated and described in detail, it should be understood that the present invention is not limited to the particular embodiments described. Various modifications, adaptations, and variations of the present invention may be made without departing from the spirit and scope of the present invention, and such modifications, adaptations, and variations are intended to be within the scope of the present invention.

## Claims

1. An antibody or an antigen binding fragment thereof capable of specifically binding to an AXL protein, comprising:
(a) a heavy chain variable region comprising the following three complementary determining regions:
(i) VH CDR1 consisting of a sequence of SEQ ID NO: 9, or a sequence having one or more amino acid substitutions, deletions, or additions compared thereto,
(ii) VH CDR2 consisting of a sequence of SEQ ID NO: 10, or a sequence having one or more amino acid substitutions, deletions, or additions compared thereto, and
(iii) VH CDR3 consisting of a sequence of SEQ ID NO: 11, or a sequence having one or more amino acid substitutions, deletions, or additions compared thereto;
and/or
(b) a light chain variable region comprising the following three complementary determining regions:
(iv) VL CDR1 consisting of a sequence of SEQ ID NO: 12, or a sequence having one or more amino acid substitutions, deletions, or additions compared thereto,
(v) VL CDR2 consisting of a sequence of SEQ ID NO: 13, or a sequence having one or more amino acid substitutions, deletions, or additions compared thereto, and
(vi) VL CDR3 consisting of a sequence of SEQ ID NO: 14, or a sequence having one or more amino acid substitutions, deletions, or additions compared thereto;
preferably, the substitution described in any one of (i)-(vi) is a conservative substitution;
and
preferably, the VH of the antibody or the antigen binding fragment thereof comprises: VH CDR1 as shown in SEQ ID NO: 9, VH CDR2 as shown in SEQ ID NO: 10, and VH CDR3 as shown in SEQ ID NO: 11; and the VL of the antibody or the antigen binding fragment thereof comprises: VL CDR1 as shown in SEQ ID NO: 12, VL CDR2 as shown in SEQ ID NO: 13, and VL CDR3 as shown in SEQ ID NO: 14.

2. An antibody or an antigen binding fragment thereof capable of specifically binding to an AXL protein, comprising:
(a) a heavy chain variable region comprising the following three complementary determining regions:
(i) VH CDR1 consisting of a sequence of SEQ ID NO: 15, or a sequence having one or more amino acid substitutions, deletions, or additions compared thereto,
(ii) VH CDR2 consisting of a sequence of SEQ ID NO: 16, or a sequence having one or more amino acid substitutions, deletions, or additions compared thereto, and
(iii) VH CDR3 consisting of a sequence of SEQ ID NO: 17, or a sequence having one or more amino acid substitutions, deletions, or additions compared thereto;
and/or
(b) a light chain variable region comprising the following three complementary determining regions:
(iv) VL CDR1 consisting of a sequence of SEQ ID NO: 18, or a sequence having one or more amino acid substitutions, deletions, or additions compared thereto,
(v) VL CDR2 consisting of a sequence of SEQ ID NO: 19, or a sequence having one or more amino acid substitutions, deletions, or additions compared thereto, and
(vi) VL CDR3 consisting of a sequence of SEQ ID NO: 20, or a sequence having one or more amino acid substitutions, deletions, or additions compared thereto;
preferably, the substitution described in any one of (i)-(vi) is a conservative substitution;
and
preferably, the VH of the antibody or the antigen binding fragment thereof comprises VH CDR1 as shown in SEQ ID NO: 15, VH CDR2 as shown in SEQ ID NO: 16, and VH CDR3 as shown in SEQ ID NO: 17; and the VL of the antibody or the antigen binding fragment thereof comprises: VL CDR1 as shown in SEQ ID NO: 18, VL CDR2 as shown in SEQ ID NO: 19, and VL CDR3 as shown in SEQ ID NO: 20.

3. An antibody or an antigen binding fragment thereof capable of specifically binding to an AXL protein, comprising:
(a) a heavy chain variable region comprising the following three complementary determining regions:
(i) VH CDR1 consisting of a sequence of SEQ ID NO: 21, or a sequence having one or more amino acid substitutions, deletions, or additions compared thereto,
(ii) VH CDR2 consisting of a sequence of SEQ ID NO: 22, or a sequence having one or more amino acid substitutions, deletions, or additions compared thereto, and
(iii) VH CDR3 consisting of a sequence of SEQ ID NO: 23, or a sequence having one or more amino acid substitutions, deletions, or additions compared thereto;
and/or
(b) a light chain variable region comprising the following three complementary determining regions:
(iv) VL CDR1 consisting of a sequence of SEQ ID NO: 24, or a sequence having one or more amino acid substitutions, deletions, or additions compared thereto,
(v) VL CDR2 consisting of a sequence of SEQ ID NO: 25, or a sequence having one or more amino acid substitutions, deletions, or additions compared thereto, and
(vi) VL CDR3 consisting of a sequence of SEQ ID NO: 26, or a sequence having one or more amino acid substitutions, deletions, or additions compared thereto;
preferably, the substitution described in any one of (i)-(vi) is a conservative substitution;
and
preferably, the VH of the antibody or the antigen binding fragment thereof comprises VH CDR1 as shown in SEQ ID NO: 21, VH CDR2 as shown in SEQ ID NO: 22, and VH CDR3 as shown in SEQ ID NO: 23; and the VL of the antibody or the antigen binding fragment thereof comprises: VL CDR1 as shown in SEQ ID NO: 24, VL CDR2 as shown in SEQ ID NO: 25, and VL CDR3 as shown in SEQ ID NO: 26.

4. An antibody or an antigen binding fragment thereof capable of specifically binding to an AXL protein, comprising:
(a) a heavy chain variable region comprising the following three complementary determining regions:
(i) VH CDR1 consisting of a sequence of SEQ ID NO: 27, or a sequence having one or more amino acid substitutions, deletions, or additions compared thereto,
(ii) VH CDR2 consisting of a sequence of SEQ ID NO: 28, or a sequence having one or more amino acid substitutions, deletions, or additions compared thereto, and
(iii) VH CDR3 consisting of a sequence of SEQ ID NO: 29, or a sequence having one or more amino acid substitutions, deletions, or additions compared thereto;
and/or
(b) a light chain variable region comprising the following three complementary determining regions:
(iv) VL CDR1 consisting of a sequence of SEQ ID NO: 30, or a sequence having one or more amino acid substitutions, deletions, or additions compared thereto,
(v) VL CDR2 consisting of a sequence of SEQ ID NO: 31, or a sequence having one or more amino acid substitutions, deletions, or additions compared thereto, and
(vi) VL CDR3 consisting of a sequence of SEQ ID NO: 32, or a sequence having one or more amino acid substitutions, deletions, or additions compared thereto;
preferably, the substitution described in any one of (i)-(vi) is a conservative substitution;
and
preferably, the VH of the antibody or the antigen binding fragment thereof comprises: VH CDR1 as shown in SEQ ID NO: 27, VH CDR2 as shown in SEQ ID NO: 28, and VH CDR3 as shown in SEQ ID NO: 29; and the VL of the antibody or the antigen binding fragment thereof comprises: VL CDR1 as shown in SEQ ID NO: 30, VL CDR2 as shown in SEQ ID NO: 31, and VL CDR3 as shown in SEQ ID NO: 32.

5. An antibody or an antigen binding fragment thereof capable of specifically binding to an AXL protein, comprising a heavy chain variable region and a light chain variable region, wherein
the heavy chain variable region comprises three CDRs contained in the heavy chain variable region shown in any one of SEQ ID NOs: 1, 3, 5, and 7; and the light chain variable region comprises three CDRs contained in the light chain variable region shown in any one of SEQ ID NOs: 2, 4, 6, and 8; and
preferably, the three CDRs contained in the heavy chain variable region and/or the three CDRs contained in the light chain variable region are defined by the Kabat, Chothia, or IMGT numbering system.

6. The antibody or the antigen binding fragment thereof of claim 1, wherein the antibody or the antigen binding fragment thereof comprises:
(a) a heavy chain variable region comprising an amino acid sequence selected from the group consisting of:
(i) a sequence as shown in SEQ ID NO: 1;
(ii) a sequence having one or more amino acid substitutions, deletions, or additions compared to the sequence as shown in SEQ ID NO: 1; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 1;
and/or,
(b) a light chain variable region comprising an amino acid sequence selected from the group consisting of:
(iv) a sequence as shown in SEQ ID NO: 2;
(v) a sequence having one or more amino acid substitutions, deletions, or additions compared to the sequence as shown in SEQ ID NO: 2; or
(vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 2;
preferably, the substitution described in in (ii) or (v) is a conservative substitution; and
preferably, the antibody or the antigen binding fragment thereof comprises: VH having the sequence as shown in SEQ ID NO: 1 and VL having the sequence as shown in SEQ ID NO: 2.

7. The antibody or the antigen binding fragment thereof of claim 2, wherein the antibody or the antigen binding fragment thereof comprises:
(a) a heavy chain variable region comprising an amino acid sequence selected from the group consisting of:
(i) a sequence as shown in SEQ ID NO: 3;
(ii) a sequence having one or more amino acid substitutions, deletions, or additions compared to the sequence as shown in SEQ ID NO: 3; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 3;
and/or,
(b) a light chain variable region comprising an amino acid sequence selected from the group consisting of:
(iv) a sequence as shown in SEQ ID NO: 4;
(v) a sequence having one or more amino acid substitutions, deletions, or additions compared to the sequence as shown in SEQ ID NO: 4; or
(vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 4;
preferably, the substitution described in in (ii) or (v) is a conservative substitution; and
preferably, the antibody or the antigen binding fragment thereof comprises: VH having the sequence as shown in SEQ ID NO: 3 and VL having the sequence as shown in SEQ ID NO: 4.

8. The antibody or the antigen binding fragment thereof of claim 3, wherein the antibody or the antigen binding fragment thereof comprises:
(a) a heavy chain variable region comprising an amino acid sequence selected from the group consisting of:
(i) a sequence as shown in SEQ ID NO: 5;
(ii) a sequence having one or more amino acid substitutions, deletions, or additions compared to the sequence as shown in SEQ ID NO: 5; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 5;
and/or,
(b) a light chain variable region comprising an amino acid sequence selected from the group consisting of:
(iv) a sequence as shown in SEQ ID NO: 6;
(v) a sequence having one or more amino acid substitutions, deletions, or additions compared to the sequence as shown in SEQ ID NO: 6; or
(vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 6;
preferably, the substitution described in in (ii) or (v) is a conservative substitution; and
preferably, the antibody or the antigen binding fragment thereof comprises: VH having the sequence as shown in SEQ ID NO: 5 and VL having the sequence as shown in SEQ ID NO:6.

9. The antibody or the antigen binding fragment thereof of claim 4, wherein the antibody or the antigen binding fragment thereof comprises:
(a) a heavy chain variable region comprising an amino acid sequence selected from the group consisting of:
(i) a sequence as shown in SEQ ID NO: 7;
(ii) a sequence having one or more amino acid substitutions, deletions, or additions compared to the sequence as shown in SEQ ID NO: 7; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 7;
and/or,
(b) a light chain variable region comprising an amino acid sequence selected from the group consisting of:
(iv) a sequence as shown in SEQ ID NO: 8;
(v) a sequence having one or more amino acid substitutions, deletions, or additions compared to the sequence as shown in SEQ ID NO: 8; or
(vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 8;
preferably, the substitution described in in (ii) or (v) is a conservative substitution; and
preferably, the antibody or the antigen binding fragment thereof comprises: VH having the sequence as shown in SEQ ID NO: 7 and VL having the sequence as shown in SEQ ID NO: 8.

10. The antibody or the antigen binding fragment thereof of any one of claims 1-9, wherein the antibody or the antigen binding fragment thereof further comprises:
(a) a heavy chain constant region of a human immunoglobulin or a variant thereof having one or more amino acid substitutions, deletions, or additions as compared to the sequence from which it is derived; and
(b) a light chain constant region of a human immunoglobulin or a variant thereof having up to 20 amino acid conservative substitutions as compared to the sequence from which it is derived;
preferably, the heavy chain constant region is an IgG heavy chain constant region, more preferably an IgG1, IgG2, IgG3, or IgG4 heavy chain constant region, further preferably, a human IgG1 or human IgG4 heavy chain constant region; and
preferably, the light chain constant region is a kappa light chain constant region.

11. The antibody or the antigen binding fragment thereof of any one of claims 1-10, wherein the antigen binding fragment is selected from Fab, Fab', (Fab')₂, Fv, disulfide-linked Fv, scFv, a diabody, and a single domain antibody; and/or the antibody is a murine antibody, a chimeric antibody, a humanized antibody, a bispecific antibody, or a multi-specific antibody; more preferably, the antibody is a fully human antibody.

12. A chimeric antigen receptor T cell comprising the antibody or the antigen binding fragment thereof of any one of claims 1-11;
preferably, the heavy chain variable region and the light chain variable region in the antibody or the antigen binding fragment are combined in tandem or in parallel.

13. An isolated nucleic acid molecule encoding the antibody or the antigen binding fragment thereof, or the heavy chain variable region and/or the light chain variable region thereof, of any one of claims 1-11.

14. A vector comprising the isolated nucleic acid molecule of claim 13;
preferably, the vector is a cloning vector or an expression vector;
more preferably, the vector is a virus;
further preferably, the viral backbone of the viral vector is derived from a modified or engineered vaccinia virus Tiantan strain, vaccinia virus New York strain, vaccinia virus Copenhagen strain, vaccinia virus canarypox strain, vaccinia virus Ankara strain, adenovirus vector, adeno-associated virus vector, herpes simplex virus vector, varicella-zoster virus vector, respiratory syncytial virus, Semliki forest virus, Epstein-Barr virus, cytomegalovirus, human herpes virus type 6, variola virus, molluscum contagiosum virus, contagious ecthyma virus, respiratory enteric orphan virus, rotavirus, enterovirus, seneca virus, poliovirus, coxsackie virus, rhinovirus, hepatitis A virus, foot and mouth disease virus, togavirus, alphavirus, eastern equine encephalitis virus, sindbis virus, rubella virus, coronavirus, flavivirus, hepatitis C virus, Japanese encephalitis virus, st. Louis encephalitis virus, murray valley fever virus, yellow fever virus, west nile virus, zika virus, dengue virus, ebola virus, marburg virus, arenavirus, lassa fever virus, lymphocytic choriomeningitis virus, picinde virus, Junin virus, Machupo virus, Hantaan virus, rift Valley fever virus, paramyxovirus, human parainfluenza virus, mumps virus, simian virus 5, measles virus, vesicular stomatitis virus, rabies virus, respiratory syncytial virus, orthomyxovirus, influenza A virus, influenza B virus, influenza C virus, hepatitis D virus, simian immunodeficiency virus, human immunodeficiency virus type 1, human immunodeficiency virus type 2, rous sarcoma virus, human T-cell lymphotropic virus type-1, simian foamy virus, hepatitis B virus, hepatitis E virus, human papilloma virus, or polyoma virus; and
more preferably, the vector is the cloning vector AbVec-hIgKappa or the cloning vector AbVec-hIgG1.

15. A host cell comprising the isolated nucleic acid molecule of claim 13 or the vector of claim 14;
preferably, the host cell is prokaryotic or eukaryotic; more preferably, the host cell is selected from *Escherichia coli* cells, yeast cells, mammalian cells or other cells suitable for the production of antibodies or antigen binding fragments, multi-specific antibodies; further preferably, the host cell is a mammalian cell; further more preferably, the host cell is a human, murine, ovine, equine, canine or feline cell; most preferably, the host cell is a 293 cell or a CHO cell.

16. A method for preparing the antibody or the antigen binding fragment thereof of any one of claims 1-11, comprising culturing the host cell of claim 15 under conditions that allow expression of the antibody or the antigen binding fragment thereof of any one of claims 1-11, and recovering the antibody or the antigen binding fragment thereof from the cultured host cell culture.

17. A bispecific or multi-specific molecule comprising the antibody or the antigen binding fragment thereof of any one of claims 1-11;
preferably, the bispecific or multi-specific molecule specifically binds to an AXL protein and additionally specifically binds to one or more other targets;
preferably, the bispecific or multi-specific molecule further comprises at least one molecule having a second binding specificity for a second target, preferably a secondary antibody; and
preferably, the bispecific or multi-specific molecule further comprises other antibodies or antigen binding fragments that specifically bind to an epitope of the AXL protein.

18. An immunoconjugate comprising the antibody or the antigen binding fragment thereof of any one of claims 1-11, and a therapeutic agent connected to the antibody or the antigen binding fragment thereof;
preferably, the therapeutic agent is selected from a cytotoxic agent;
preferably, the therapeutic agent is selected from an alkylating agent, a mitotic inhibitor, an antitumor antibiotic, an antimetabolite, a topoisomerase inhibitor, a tyrosine kinase inhibitor, a radionuclide agent, and any combination thereof; and
preferably, the immunoconjugate is an antibody-drug conjugate.

19. A pharmaceutical composition comprising the antibody or the antigen binding fragment thereof of any one of claims 1-11, the bispecific or multi-specific molecule of claim 17, or the immunoconjugate of claim 18, and a pharmaceutically acceptable carrier and/or excipient;
preferably, the pharmaceutical composition further comprises an additional pharmaceutically active agent;
preferably, the additional pharmaceutically active agent is a drug having anti-tumor activity; more preferably the pharmaceutically active agent is an alkylating agent, a mitotic inhibitor, an antitumor antibiotic, an antimetabolite, a topoisomerase inhibitor, a tyrosine kinase inhibitor, a radionuclide agent, a radiosensitizer, an antiangiogenic agent, a cytokine, a molecularly targeted drug, an immune checkpoint inhibitor, or an oncolytic virus; and
preferably, the antibody or the antigen binding fragment thereof, the bispecific or multi-specific molecule or the immunoconjugate, and the additional pharmaceutically active agent are provided as separate components or as components of the same composition.

20. A kit comprising the antibody or the antigen binding fragment thereof of any one of claims 1-11;
preferably, the antibody or the antigen binding fragment thereof carries a detectable label, a radionuclide, a fluorescent dye, a luminescent substance, or a biotin; more preferably, the detectable label is an enzyme, further preferably horseradish peroxidase; more preferably, the luminescent substance is a chemiluminescent substance;
preferably, the kit further comprises a secondary antibody that specifically recognizes the antibody or the antigen binding fragment thereof of any one of claims 1-11; and
preferably, the secondary antibody further comprises a detectable label, a radionuclide, a fluorescent dye, a luminescent substance, or a biotin; more preferably, the detectable label is an enzyme, further preferably horseradish peroxidase; more preferably, the luminescent substance is a chemiluminescent substance.

21. A chimeric antigen receptor comprising an antigen binding domain of the antibody or the antigen binding fragment thereof of any one of claims 1-11;
preferably, the antigen binding domain comprises a heavy chain variable region and a light chain variable region of the antibody or the antigen binding fragment thereof of any one of claims 1-11;
preferably, the antigen binding domain is scFv;
preferably, the antigen binding receptor comprises an antigen binding fragment of the antibody of any one of claims 1-11;
preferably, the antigen binding receptor is expressed by an immune effector cell;
more preferably, the immune effector cell is a T cell.

22. An isolated nucleic acid molecule encoding the chimeric antigen receptor of claim 21.

23. A vector comprising the isolated nucleic acid molecule of claim 22; preferably, it being used to prepare a chimeric antigen receptor T cell.

24. A host cell comprising the isolated nucleic acid molecule of claim 22 or the vector of claim 23;
preferably, the host cell is an immune effector cell; more preferably, the immune effector cell is a T cell or an NK cell; and
preferably, the host cell is a chimeric antigen receptor T cell.

25. A method for inhibiting tumor cell growth and/or killing the tumor cell, comprising contacting the tumor cell with an effective amount of the antibody or the antigen binding fragment thereof of any one of claims 1-11, the bispecific or multi-specific molecule of claim 17, the immunoconjugate of claim 18, the pharmaceutical composition of claim 19, the chimeric antigen receptor of claim 21, or the host cell of claim 24.

26. A method for preventing and/or treating a tumor in a subject, comprising administering to the subject in need thereof an effective amount of the antibody or the antigen binding fragment thereof of any one of claims 1-11, the bispecific or multi-specific molecule of claim 17, the immunoconjugate of claim 18, the pharmaceutical composition of claim 19, the chimeric antigen receptor of claim 21, or the host cell of claim 24;
preferably, the subject is a human;
preferably, the tumor is selected from B-cell lymphoma, T-cell lymphoma, melanoma, prostate cancer, renal cell carcinoma, sarcoma, glioma, preferably high-grade glioma, blastoma, preferably neuroblastoma, osteosarcoma, plasmacytoma, histiocytoma, pancreatic cancer, breast cancer, lung cancer, preferably small cell lung cancer and non-small cell lung cancer, gastric cancer, liver cancer, colon cancer, rectal cancer, esophageal cancer, large intestine cancer, hematopoietic system cancer, testicular cancer, cervical cancer, ovarian cancer, bladder cancer, squamous cell cancer, adenocarcinoma, AIDS-related lymphoma, bladder cancer, brain cancer, cancer of the nervous system, head and neck cancer, squamous cell carcinoma of the head and neck, Hodgkin's lymphoma, non-Hodgkin' s lymphoma, or blood-borne neoplastic disease;
preferably, the subject is a mammal, more preferably a human;
preferably, the method further comprises administering an additional agent having anti-tumor activity; more preferably the additional agent having anti-tumor activity is an alkylating agent, a mitotic inhibitor, an antitumor antibiotic, an antimetabolite, a topoisomerase inhibitor, a tyrosine kinase inhibitor, a radionuclide, a radiosensitizer, an antiangiogenic agent, a cytokine, a molecularly targeted drug, an immune checkpoint inhibitor, or an oncolytic virus;
preferably, the method further comprises administering an additional anti-tumor therapy; more preferably, the additional anti-tumor therapy is surgery, chemotherapy, radiation therapy, targeted therapy, immunotherapy, hormonal therapy, gene therapy, or palliative therapy.

27. Use of the antibody or the antigen binding fragment thereof of any one of claims 1-11, the bispecific or multi-specific molecule of claim 17, the immunoconjugate of claim 18, the pharmaceutical composition of claim 19, the chimeric antigen receptor of claim 21, or the host cell of claim 24, in the preparation of a medicament for the prevention and/or treatment of a tumor in a subject;
preferably, the subject is a human;
preferably, the medicament further comprises an additional pharmaceutically active agent;
preferably, the additional pharmaceutically active agent is an agent having anti-tumor activity; more preferably the pharmaceutically active agent is an alkylating agent, a mitotic inhibitor, an antitumor antibiotic, an antimetabolite, a topoisomerase inhibitor, a tyrosine kinase inhibitor, a radionuclide agent, a radiosensitizer, an antiangiogenic agent, a cytokine, a molecularly targeted drug, an immune checkpoint inhibitor, or an oncolytic virus; and
preferably, the tumor is selected from B-cell lymphoma, T-cell lymphoma, melanoma, prostate cancer, renal cell carcinoma, sarcoma, glioma, preferably high-grade glioma, blastoma, preferably neuroblastoma, osteosarcoma, plasmacytoma, histiocytoma, pancreatic cancer, breast cancer, lung cancer, preferably small cell lung cancer and non-small cell lung cancer, gastric cancer, liver cancer, colon cancer, rectal cancer, esophageal cancer, large intestine cancer, hematopoietic system cancer, testicular cancer, cervical cancer, ovarian cancer, bladder cancer, squamous cell cancer, adenocarcinoma, AIDS-related lymphoma, bladder cancer, brain cancer, cancer of the nervous system, head and neck cancer, squamous cell carcinoma of the head and neck, Hodgkin's lymphoma, non-Hodgkin' s lymphoma, or blood-borne neoplastic disease; and
preferably, the subject is a mammal, more preferably a human.
